# EUROPEAN PATENT APPLICATION

(11) **EP 4 516 782 A1**
(43) Date of publication of application: **05.03.2025**
(21) Application number: 23795565.3
(22) Date of filing: 27.04.2023
(51) Int. Cl.: C07D 307/14, A61K 31/341, A61P 25/28

(54) **CRYSTAL FORM OF BLARCAMESINE HYDROCHLORIDE, METHOD FOR PREPARING SAME, AND USE THEREOF**

(30) Priority: 29.04.2022 CN 202210472873; 29.04.2022 CN 202210473993; 02.06.2022 CN 202210623900
(71) Applicant: Crystal Pharmaceutical (Suzhou) Co., Ltd., Suzhou, Jiangsu 215123 (CN); Moehs Iberica SL, 08191 Rubi, Barcelona (ES)
(72) Inventor: ZHANG, Yuxing, Suzhou, Jiangsu 215123 (CN); ZHU, Hongyan, Suzhou, Jiangsu 215123 (CN); MENG, Liping, Suzhou, Jiangsu 215123 (CN)
(74) Representative: dompatent von Kreisler Selting Werner - Partnerschaft von Patent- und Rechtsanwälten mbB
(86) International application number: PCT/CN2023/091293
(87) International publication number: WO 2023/208133

(57) **Abstract**

The present disclosure relates to novel crystalline forms of Blarcamesine hydrocholoride (hereinafter referred to as "compound I"), a method for preparing same, a pharmaceutical composition comprising the crystalline forms, and use of the crystalline forms in preparing a Sigma-1 receptor agonist and a medicament for treating Rett syndrome, Alzheimer's disease, and Parkinson's disease dementia.

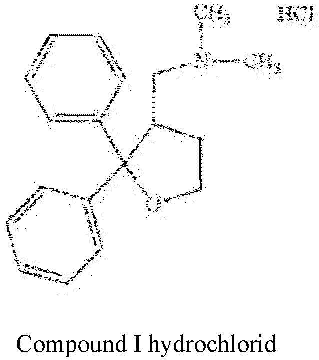

## Description

### TECHNICAL FIELD

The present disclosure pertains to the field of chemical crystallography, particularly relates to novel crystalline forms of Blarcamesine hydrochloride preparation method and use thereof.

### BACKGROUND

Alzheimer's disease is a degenerative brain disorder and the most common form of dementia. It is an irreversible progressive brain disease that slowly destroys memory and thinking skills, eventually impairing the ability to carry out even the simplest tasks. Experts predict that by 2050, the global number of dementia cases is expected to exceed 130 million, thus creating a significant demand for medications.

Parkinson's disease is a neurodegenerative disorder involving damaged protein clearance, mitochondrial impairment, and brain inflammation. Over half of Parkinson's disease patients may develop Parkinson's disease dementia. Parkinson's disease is progressive and can lead to many cognitive and behavioral deficits, including anxiety, difficulty with thinking and speaking, sleep disturbances, and dementia. Globally, over 10 million people have been diagnosed with Parkinson's disease, ranking it as the 14th leading cause of death.

Rett syndrome is a rare genetic neurological disorder first observed in infancy. Rett syndrome causes severe damage, affecting nearly every aspect of a person's life. Associated symptoms include respiratory and sleep abnormalities, seizures, spinal curvature, abnormal muscle tone, and gastrointestinal issues. There are approximately 350,000 Rett syndrome patients worldwide, making it the second most common cause of severe intellectual disability in females. However, there are currently no drugs on the market specifically for treating Rett syndrome.

It is noteworthy that Blarcamesine, an oral Sigma-1 receptor agonist developed by Anavex Life Sciences, is undergoing clinical trials for Rett syndrome, early-stage Alzheimer's disease, and Parkinson's disease dementia, showing promising clinical results. The chemical name of Blarcamesine is tetrahydro-N,N-dimethyl-2,2-diphenyl-3-furanmethanamine (referred to as Compound I), and the structure of Compound I hydrochloride is shown as the follows:

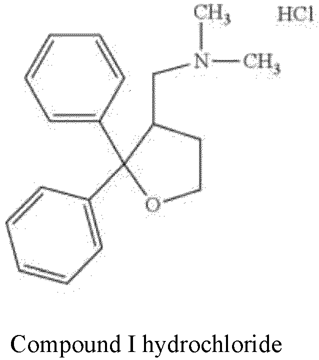

In the development of small molecule drugs, drug polymorphism is a common phenomenon in drug development and an important factor affecting drug quality. A crystalline form is a solid formed by the three-dimensional ordered arrangement of compound molecules in a microstructure to form a lattice. Polymorphism refers to the phenomenon that a compound exists in multiple crystalline forms. Compounds may exist in one or more crystalline forms, but their existence and characteristics cannot be specifically predicted. Different crystalline forms of drug substances have different physicochemical properties, which can affect drug's in vivo dissolution and absorption and will further affect drug's clinical efficacy and safety to some extent. Especially for some poorly soluble oral solid or semi-solid dosage forms, crystalline forms are crucial to product performance. In addition, drug substances with different polymorphs have varying manufacturability, such as compressibility, flowability, and so on. These properties may affect the drug mixing and tableting processes. Therefore, polymorphism is an important part of drug research and drug quality control. Therefore, comprehensive studies on the crystallization behavior of Compound I hydrochloride are needed to achieve crystalline forms that meet the pharmaceutical requirements of Compound I, including acceptable physicochemical properties such as chemical stability, thermal stability, solubility, hygroscopicity, and/or particle size; manufacturability, including yield, impurity exclusion during crystallization, filtration properties, drying properties, and milling properties; and formulation feasibility, including crystalline stability relative to pressure or compression force during tableting. Prior art WO2021158586A1 disclosed an n-butanol solvate Form B3 of Compound I hydrochloride. Currently, the FDA's Inactive Ingredient Database (IID) does not include maximum daily intake and maximum daily exposure information for n-butanol across all routes of administration, thus no drug using n-butanol solvate has been marketed in the United States.

Prior art WO2017013498A1 disclosed three forms of Compound I hydrochloride, namely Form I, Form II, and Form III. Forms I, II and III all require preparation under high-pressure conditions using supercritical fluids, making their production processes complex and difficult to scale up. Additionally, XRPD patterns from this prior art reveal that Forms I and II contain significant amorphous, which can affect the stability, processing, storage, hygroscopicity, and dissolution rates of the active pharmaceutical ingredient. Moreover, SEM images indicate that Form III forms fine needle-like crystals, which may exhibit strong electrostatic adsorption, making filtration challenging and scaling up production difficult.

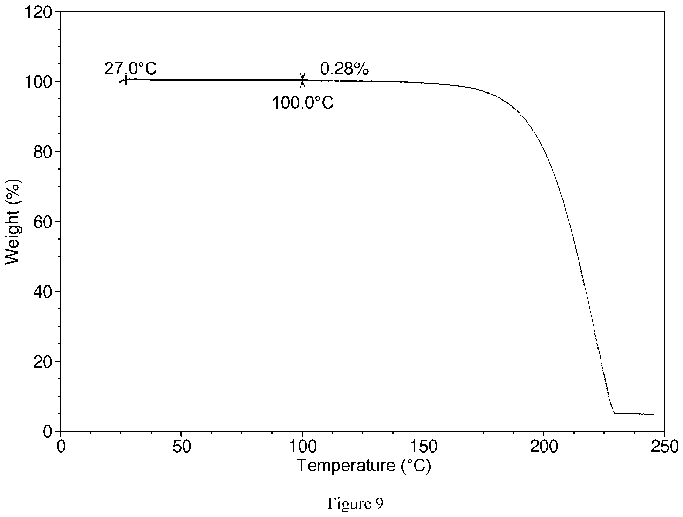

Prior art WO2019200345A1 disclosed eight forms of Compound I hydrochloride, namely Forms I, II, III, IV, V, VI, VII and VIII. Paragraph 0085 of the disclosure notes that Form II is hygroscopic and prone to variation in water content. Paragraph 0093 notes that Form IV is a mixture containing amorphous. As shown in the XRPD pattern of Figure 9, it contains a high amount of amorphous material. Paragraph 0099 describes Form VIII as a trihydrate prone to rapid dehydration into Form I. Furthermore, paragraph 0081 specifies that Form I is the thermodynamically preferred polymorph and is currently used in clinical stages. Therefore, Form I as disclosed in WO2019200345A1 (referred to hereafter as "prior art Form I") represents the polymorph with the most optimal properties among those disclosed in prior arts.

Further research by the inventors has revealed that the powder properties of Form I are not ideal (e.g., poor flowability, compressibility), with high hygroscopicity and poor solubility in common organic solvents. These issues may lead to uneven dispersion during product blending, difficulties in tablet compression such as poor formation, cracking, and crumbling, resulting in variations in the active pharmaceutical ingredient content among particles within the same batch. Poor uniformity in content or significant differences in weight may cause fluctuations in the intake of the active pharmaceutical ingredient at different times for different patients or the same patient, affecting drug absorption and blood concentration, thus impacting therapeutic efficacy.

To overcome the disadvantages of prior arts, a novel crystalline form meeting the medicinal standard is still needed for the development of drugs containing Compound I hydrochloride.

After a careful research and extensive experiments, the inventors of the present disclosure surprisingly discovered crystalline forms of Compound I hydrochloride. On the basis of balancing good stability, they have advantages in at least one aspect of solubility, hygroscopicity, purification ability, stability, adhesiveness, compressibility, flowability, in vitro and in vivo dissolution, and bioavailability, etc. In particular, they have advantages such as no solvent residue, high solubility in organic solvents, high in vitro dissolution, low hygroscopicity, good purification ability, good flowability, good compressibility, which solves the problems existing in prior arts and is of great significance for the development of drugs containing Compound I.

### SUMMARY

The present disclosure is to provide novel crystalline forms of Compound I hydrochloride, preparation method, and pharmaceutical compositions comprising crystalline forms.

According to the objective of the present disclosure, a tartaric acid co-crystal of Compound I hydrochloride is provided.

According to the objective of the present disclosure, tartaric acid co-crystal form CSII of Compound I hydrochloride is provided (hereinafter referred to as Form CSII).

In one aspect provided herein, the X-ray powder diffraction pattern of Form CSII comprises characteristic one or two or three peaks at 2theta values of 8.6°±0.2°, 12.8°±0.2° and 20.5°±0.2° using CuKα radiation. Preferably, the X-ray powder diffraction pattern of Form CSII comprises characteristic peaks at 2theta values of 8.6°±0.2°, 12.8°±0.2° and 20.5°±0.2° using CuKα radiation.

Furthermore, the X-ray powder diffraction pattern of Form CSII comprises characteristic one or two or three peaks at 2theta values of 14.7°±0.2°, 21.1°±0.2° and 21.8°±0.2° using CuKα radiation. Preferably, the X-ray powder diffraction pattern of Form CSII comprises characteristic peaks at 2theta values of 14.7°±0.2°, 21.1°±0.2° and 21.8°±0.2° using CuKα radiation.

Furthermore, the X-ray powder diffraction pattern of Form CSII comprises characteristic one or two or three peaks at 2theta values of 13.5°±0.2°, 15.6°±0.2° and 17.5°±0.2° using CuKα radiation. Preferably, the X-ray powder diffraction pattern of Form CSII comprises characteristic peaks at 2theta values of 13.5°±0.2°, 15.6°±0.2° and 17.5°±0.2° using CuKα radiation.

In another aspect provided herein, the X-ray powder diffraction pattern of Form CSII comprises one or two or three or four or five or six or seven or eight or nine or ten or eleven characteristic peaks at 2theta values of 8.6°±0.2°, 12.8°±0.2°, 20.5°±0.2°, 14.7°±0.2°, 21.1°±0.2°, 21.8°±0.2°, 13.5°±0.2°, 15.6°±0.2°, 17.5°±0.2°, 5.1°±0.2° and 6.8°±0.2° using CuKα radiation.

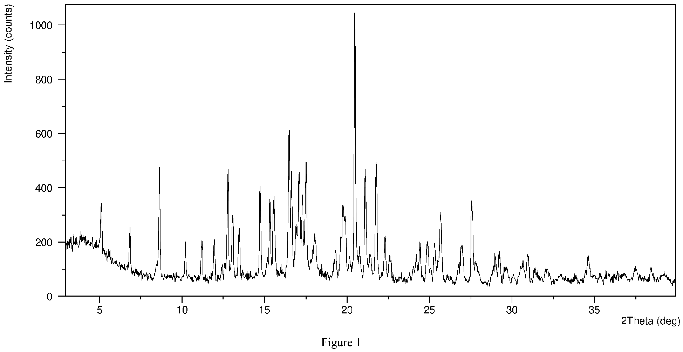

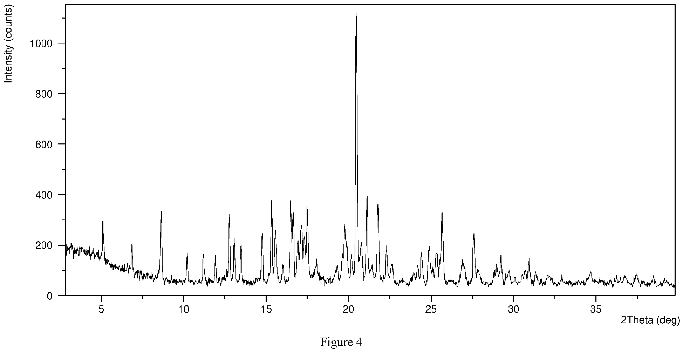

Without any limitation being implied, the XRPD pattern of Form CSII is substantially as depicted in Figure 1 or Figure 4 using CuKα radiation.

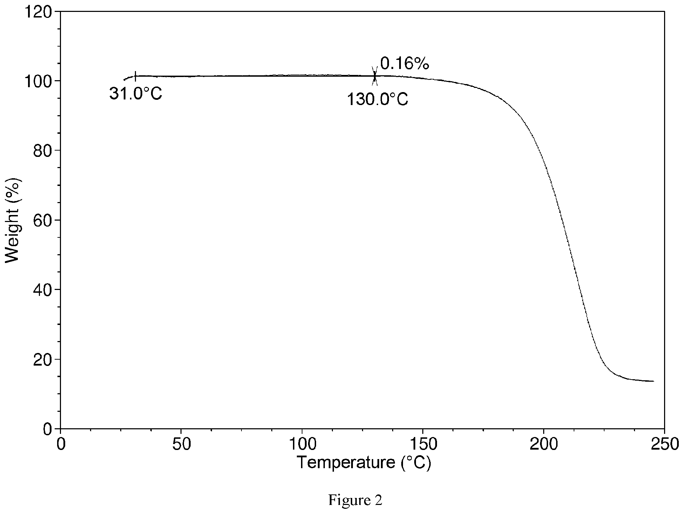

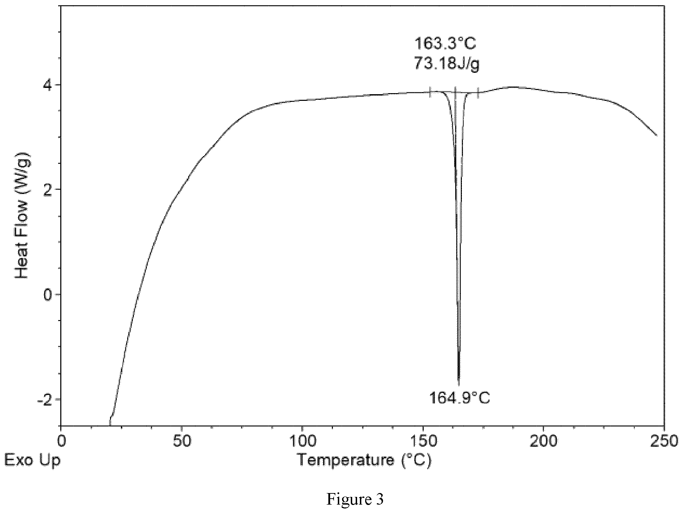

Without any limitation being implied, the TGA curve of Form CSII is substantially as depicted in Figure 2, which shows about 0.2% weight loss when heated to 130 °C. Without any limitation being implied, the DSC curve of Form CSII is substantially as depicted in Figure 3, which shows an endothermic peak. The endothermic peak is at about 163 °C (onset temperature), corresponding to a melting endothermic peak. Without any limitation being implied, Form CSII is an L-tartaric acid co-crystal of Compound I hydrochloride.

Without any limitation being implied, the molar ratio of Compound I hydrochloride to L-tartaric acid in Form CSII is 1:0.5.

Without any limitation being implied, Form CSII is an anhydrate.

According to the objective of the present disclosure, a process for preparing Form CSII is also provided. The process comprises: (1) mixing Compound I hydrochloride, tartaric acid, and a ketone solvent, grinding, then suspending and stirring the mixture in a ketone solvent for a period of time, and separating to obtain Form CSII; or (2) placing Compound I hydrochloride, Form CSII seeds, and tartaric acid in an ether solvent, suspending and stirring for a period of time, separating the solid to obtain Form CSII. Further, the feeding ratio of Compound I hydrochloride to tartaric acid is preferably 1:0.4-1:2.5. Said ketone solvent is preferably a C3-C8 ketone, more preferably methyl isobutyl ketone. Said ether solvent is preferably a C3-C8 ether, more preferably 2-methyltetrahydrofuran. Said stirring time is preferably greater than 0.5 hours. Said tartaric acid is preferably L-tartaric acid.

According to the objective of the present disclosure, a citric acid co-crystal of Compound I hydrochloride is provided.

According to the objective of the present disclosure, citric acid co-crystal form CSIII of Compound I hydrochloride is provided (hereinafter referred to as Form CSIII).

In one aspect provided herein, the X-ray powder diffraction pattern of Form CSIII comprises characteristic one or two or three peaks at 2theta values of 16.6°±0.2°, 20.2°±0.2° and 21.1°±0.2° using CuKα radiation. Preferably, the X-ray powder diffraction pattern of Form CSIII comprises characteristic peaks at 2theta values of 16.6°±0.2°, 20.2°±0.2° and 21.1°±0.2° using CuKα radiation.

Furthermore, the X-ray powder diffraction pattern of Form CSIII comprises characteristic one or two or three peaks at 2theta values of 4.3°±0.2°, 11.1°±0.2° and 12.9°±0.2° using CuKα radiation. Preferably, the X-ray powder diffraction pattern of Form CSIII comprises characteristic peaks at 2theta values of 4.3°±0.2°, 11.1°±0.2° and 12.9°±0.2° using CuKα radiation.

In another aspect provided herein, the X-ray powder diffraction pattern of Form CSIII comprises one or two or three or four or five or six or seven or eight or nine or ten or eleven characteristic peaks at 2theta values of 16.6°±0.2°, 20.2°±0.2°, 21.1°±0.2°, 4.3°±0.2°, 11.1°±0.2°, 12.9°±0.2°, 15.2°±0.2°, 17.7°±0.2°, 21.8°±0.2°, 23.0°±0.2° and 27.1°±0.2° using CuKα radiation.

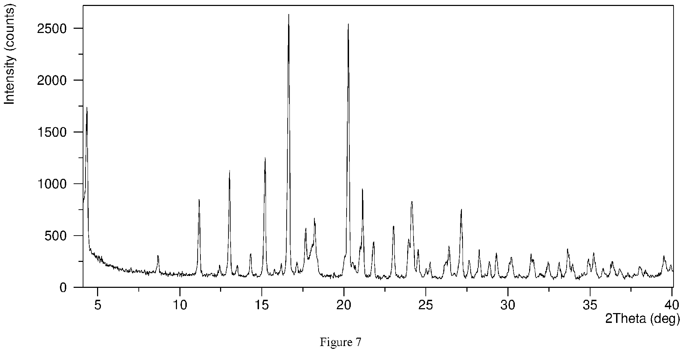

Without any limitation being implied, the XRPD pattern of Form CSIII is substantially as depicted in Figure 7 using CuKα radiation.

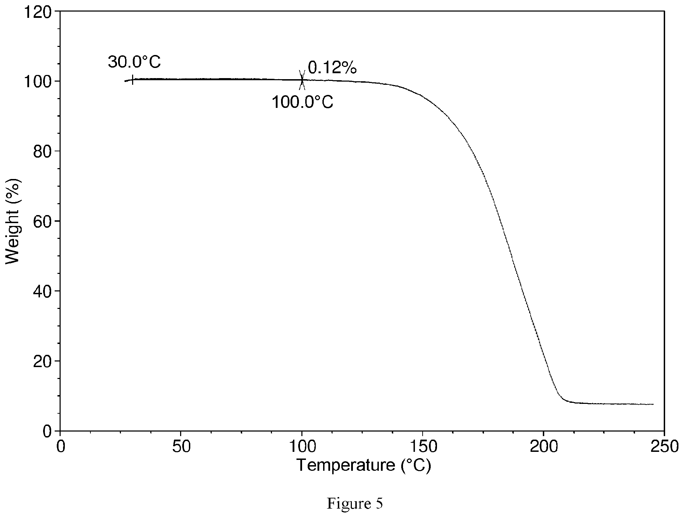

Without any limitation being implied, the TGA curve of Form CSIII is substantially as depicted in Figure 5, which shows about 0.1% weight loss when heated to 100 °C.

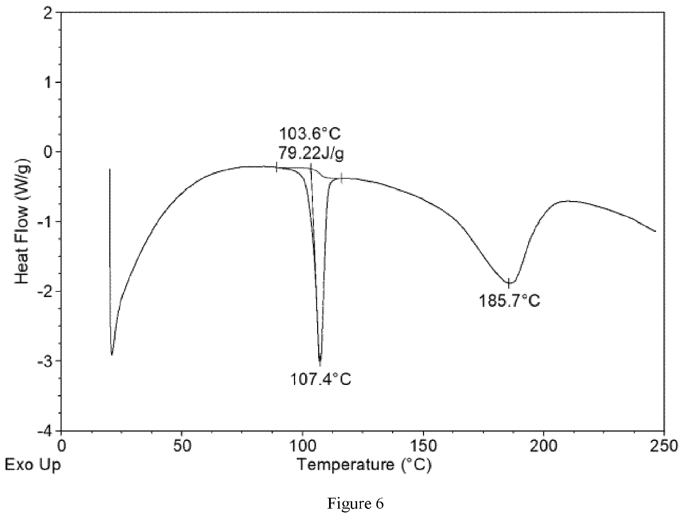

Without any limitation being implied, the DSC curve of Form CSIII is substantially as depicted in Figure 6, which shows two endothermic peaks. The first endothermic peak is at about 104 °C (onset temperature), and the second endothermic peak is at about 186 °C.

Without any limitation being implied, the molar ratio of Compound I hydrochloride to citric acid in Form CSIII is 1:1.

Without any limitation being implied, Form CSIII is an anhydrate.

According to the objective of the present disclosure, a process for preparing Form CSIII is also provided. The process comprises: (1) mixing Compound I hydrochloride, citric acid, and a ketone solvent, grinding, then suspending and stirring the mixture in a ketone solvent for a period of time, separating the solid to obtain Form CSIII; or (2) placing Compound I hydrochloride, Form CSIII seeds, and citric acid in a ketone solvent, suspending and stirring for a period of time, separating the solid to obtain Form CSIII.

Further, the feeding ratio of Compound I hydrochloride to citric acid is preferably 1:0.8-1:2. Said ketone solvent is preferably a C3-C8 ketone, more preferably methyl isobutyl ketone. Said stirring time is preferably greater than 0.5 hours.

According to the objective of the present disclosure, a malic acid co-crystal of Compound I hydrochloride is provided.

According to the objective of the present disclosure, malic acid co-crystal form CSIV of Compound I hydrochloride is provided (hereinafter referred to as Form CSIV).

In one aspect provided herein, the X-ray powder diffraction pattern of Form CSIV comprises characteristic one or two or three peaks at 2theta values of 8.6°±0.2°, 20.5°±0.2° and 21.8°±0.2° using CuKα radiation. Preferably, the X-ray powder diffraction pattern of Form CSIV comprises characteristic peaks at 2theta values of 8.6°±0.2°, 20.5°±0.2° and 21.8°±0.2° using CuKα radiation.

Furthermore, the X-ray powder diffraction pattern of Form CSIV comprises characteristic one or two or three peaks at 2theta values of 12.7°±0.2°, 11.1°±0.2° and 17.5°±0.2° using CuKα radiation. Preferably, the X-ray powder diffraction pattern of Form CSIV comprises characteristic peaks at 2theta values of 12.7°±0.2°, 11.1°±0.2° and 17.5°±0.2° using CuKα radiation.

Furthermore, the X-ray powder diffraction pattern of Form CSIV comprises characteristic one or two or three peaks at 2theta values of 6.8°±0.2°, 10.2°±0.2° and 21.0°±0.2° using CuKα radiation. Preferably, the X-ray powder diffraction pattern of Form CSIV comprises characteristic peaks at 2theta values of 6.8°±0.2°, 10.2°±0.2° and 21.0°±0.2° using CuKα radiation.

In another aspect provided herein, the X-ray powder diffraction pattern of Form CSIV comprises one or two or three or four or five or six or seven or eight or nine or ten or eleven or twelve or thirteen or fourteen or fifteen or sixteen characteristic peaks at 2theta values of 8.6°±0.2°, 20.5°±0.2°, 21.8°±0.2°, 12.7°±0.2°, 11.1°±0.2°, 17.5°±0.2°, 6.8°±0.2°, 10.2°±0.2°, 21.0°±0.2°, 15.6±0.2°, 15.3±0.2°, 13.4±0.2°, 14.6±0.2°, 22.2±0.2°, 25.7±0.2° and 27.4±0.2° using CuKα radiation.

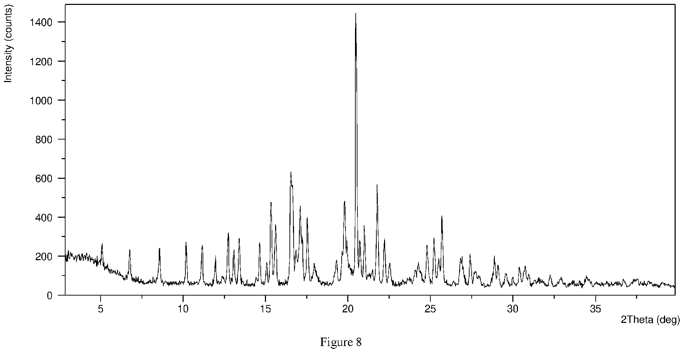

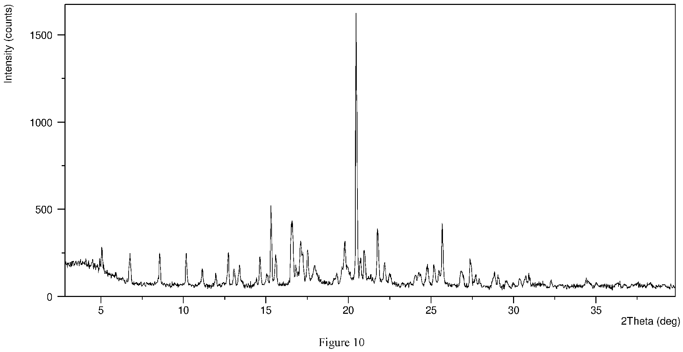

Without any limitation being implied, the XRPD pattern of Form CSIV is substantially as depicted in Figure 8 or Figure 10 using CuKα radiation.

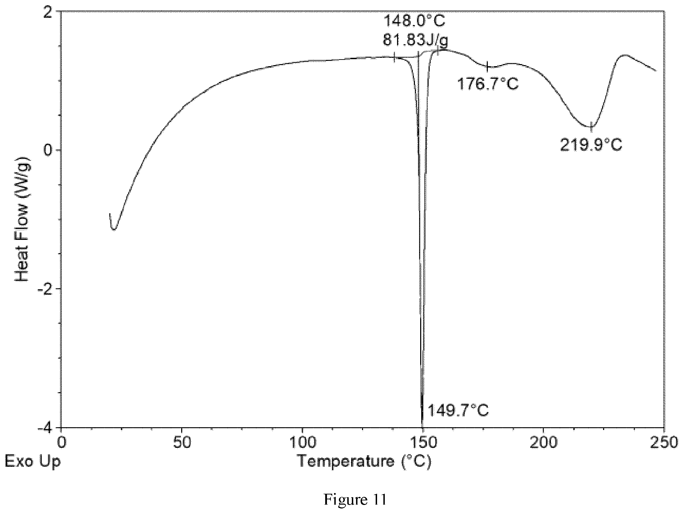

Without any limitation being implied, the TGA curve of Form CSIV is substantially as depicted in Figure 9, which shows about 0.3% weight loss when heated to 100 °C. Without any limitation being implied, the DSC curve of Form CSIV is substantially as depicted in Figure 11, which shows three endothermic peaks. The first endothermic peak is at about 148 °C (onset temperature), the second endothermic peak is at about 177 °C, and the third endothermic peak at is about 220 °C.

Without any limitation being implied, Form CSIV is a DL-malic acid co-crystal of Compound I hydrochloride.

Without any limitation being implied, the molar ratio of Compound I hydrochloride to DL-malic acid in Form CSIV is 1:0.5.

Without any limitation being implied, Form CSIV is an anhydrate.

According to the objective of the present disclosure, a process for preparing Form CSIV is also provided. The process comprises: mixing Compound I hydrochloride, malic acid, and a ketone solvent, suspending and stirring, then separating to obtain Form CSIV. Further, the feeding ratio of Compound I hydrochloride to malic acid is preferably 1:0.5-1:2. Said ketone solvent is preferably a C3-C8 ketone, more preferably methyl isobutyl ketone. Said stirring time is preferably greater than 0.5 hours.

According to the objective of the present disclosure, a pharmaceutical composition is provided, which includes a therapeutically effective amount of Form CSII, Form CSIII, Form CSIV, or any combination of these three forms, along with pharmaceutically acceptable excipients.

According to the objective of the present disclosure, the use of Form CSII, Form CSIII, Form CSIV or any combination of these three forms in the preparation of a Sigma-1 receptor agonist drug is provided.

According to the objective of the present disclosure, the use of Form CSII, Form CSIII, Form CSIV or any combination of these three forms in the preparation of drugs for treating Rett syndrome, Alzheimer's disease, and/or Parkinson's disease dementia.

### Beneficial effects and technical problem solved by the present disclosure

The technical problem addressed by the present disclosure is to provide a novel crystalline form different from prior arts. The crystalline form has no solvent residue. While maintaining excellent stability and a good dissolution base, the crystalline form has higher solubility, lower hygroscopicity, better flowability, and superior compressibility compared to prior art Form I, thus solving the problems present in prior arts.

Form CSII of the present disclosure has the following beneficial effects:
(1) Compared with prior art Form I, Form CSII of the present disclosure has lower hygroscopicity. The weight gain of prior art Form I at 70%RH is 2.3 times that of Form CSII of the present disclosure. In one aspect, high hygroscopicity tends to cause chemical degradation and polymorph transformation, which affects the physicochemical stability of the drug substance. In addition, high hygroscopicity will reduce the flowability of the drug substance, thereby affecting the processing of the drug substance. In another aspect, drug substance with high hygroscopicity requires low humidity environment during production and storage, which puts strict requirements on production and imposes higher costs. More importantly, high hygroscopicity is likely to cause variation in the content of active pharmaceutical ingredients in the drug product, thus affecting drug product quality. Form CSII provided by the present disclosure with low hygroscopicity is not demanding on the production and storage conditions, which reduces the cost of production, storage and quality control, and has strong economic value.
(2) Compared with prior art Form I, Form CSII of the present disclosure has better compressibility. Failure in hardness/friability test and tablet crack issue can be avoided due to better compressibility, making the preparation process more reliable, improving product appearance, promoting product quality and production efficiency.
(3) Compared with prior art Form I, Form CSII of the present disclosure has higher solubility in common alcohols, ketones and ethers. Especially in n-propanol, the solubility of Form CSII is at least 2-4 times that of prior art Form I. Form CSII of the present disclosure exhibits higher solubility in common organic solvents, which is beneficial for production and preparation. It also reduces the solvent volume required in the production process, making it more energy-efficient and environmentally friendly.
(4) Form CSII of the present disclosure has no residual solvent. Residual solvents can affect drug safety, quality and stability, potentially leading to crystalline transformation or impurity formation during drug's production and storage, which can affect drug bioavailability and toxicity. The absence of residual solvents in Form CSII effectively addresses issues related to low drug stability, poor efficacy, and high toxicity caused by low drug purity or high solvent residues.
(5) Form CSII of the present disclosure has good purification effect. The purity is significantly increased after the starting material is converted into Form CSII. In a specific embodiment of the present disclosure, the purity of the starting material used is 99.14%. The purity of Form CSII made from the starting material is 99.79%. The purity is increased over 0.65%. Chemical purity is of great significance for ensuring drug efficacy, safety and preventing the occurrence of adverse effects. Drug regulations have strict requirements on impurity content. As Form CSII of the present disclosure has good purification ability and is excellent in removing impurities in the crystallization process. Thus drug substances with high purity can be obtained through crystallization, which effectively overcome the disadvantages of poor stability, poor efficacy and high toxicity caused by low purity drug substances.
(6) Form CSII of the present disclosure has good physical stability under mechanical force. The crystalline state of Form CSII doesn't change after tableting, grinding and formulation process. Grinding and pulverization are often required in the drug manufacturing process. Good physical stability of the drug substance can reduce the risk of crystallinity decrease and crystal transformation during the drug manufacturing process. In addition, Form CSII has good physical stability under different pressures, which is beneficial to keep crystalline form unchanged during tableting process.
(7) Form CSII of the present disclosure has good photostability. When Form CSII is exposed to light under certain conditions for at least two weeks, it remains unchanged, and its purity remains relatively stable. Light can accelerate drug oxidation, cause photodegradation, and lead to color changes and precipitation, which not only reduces the drug's potency but also severely affects its quality and can even increase toxicity. Therefore, the stability of drug substance under light is crucial for drug. Form CSII has good photostability, which helps prevent impacts on drug quality due to crystal transformation or purity degradation during storage.
(8) From CSII drug substance and drug product of the present disclosure have good stability. Crystalline state of Form CSII drug substance doesn't change for at least 9 months when stored under the condition of 25 °C/60%RH. The chemical purity remains substantially unchanged during storage. After Form CSII is mixed with the excipients to form a drug product and stored under the condition of 25 °C/60%RH, crystalline state of Form CSII drug product doesn't change for at least one month. These results show that From CSII drug substance and drug product of the present disclosure have good stability under long term condition, which is beneficial to the drug storage. Meanwhile, crystalline state of Form CSII drug substance doesn't change for at least 6 months when stored under the condition of 40 °C/75%RH. The crystalline state of Form CSII drug substance doesn't change for at least one month when stored under the condition of 60 °C/75%RH. The chemical purity is above 99.9% and remains substantially unchanged during storage. These results show that Form CSII drug substance has good stability under accelerated and stress conditions. Drug substance and drug product would go through high temperature and high humidity conditions caused by different season, regional climate and environment during storage, transportation, and manufacturing processes. Therefore, good stability under accelerated and stress conditions is of great importance to the drug development. Form CSII drug substance and drug product have good stability under stress condition, which is beneficial to avoid the impact on drug quality due to crystal transformation or decrease in purity during drug storage.
   Good physical and chemical stability of drug substance ensure that no crystal transformation or impurities is generated during production and storage. Form CSII has good physical and chemical stability, ensuring consistent and controllable quality of the drug substance and drug product, minimizing quality change, bioavailability change and toxicity due to crystal transformation or impurity generation.
(9) Form CSII drug product of the present disclosure has good in vitro dissolution. In pH 6.8 PBS, the dissolution of Form CSII drug product at 10 minutes is up to 97.0%. Drug dissolution is a prerequisite for drug absorption. Drugs with different crystalline forms may lead different in vivo dissolution, which ultimately leads to different clinical efficacy. According to BCS guidelines, in vitro dissolution may be relevant to the prediction of in vivo performance of drug products. Good in vitro dissolution of Form CSII drug products provided by the present disclosure may leads to higher in vivo absorption, and better in vivo exposure, thereby improving drug's bioavailability and efficacy. Higher intrinsic dissolution rate of Form CSII drug substance is beneficial for the drug to achieve peak concentration in plasma quickly after administration, thus ensuring rapid drug action.

Form CSIII of the present disclosure has the following beneficial effects:
(1) Compared with prior art Form I, Form CSIII of the present disclosure has better compressibility. Failure in hardness/friability test and tablet crack issue can be avoided due to better compressibility, making the preparation process more reliable, improving product appearance, promoting product quality and production efficiency.
(2) Compared with prior art Form I, Form CSIII of the present disclosure has better flowability. Flowability evaluation results indicate that the flowability of Form CSIII is remarkably better than that of prior art forms. Better flowability can prevent clogging of production equipment and increase manufacturing efficiency. Better flowability of Form CSIII ensures the content uniformity of the drug product, reduces the weight variation of the drug product and improves product quality.
(3) Compared with prior art Form I, Form CSIII of the present disclosure has higher solubility in common alcohols, esters and ethers. Especially in tetrahydrofuran, the solubility of Form CSIII is at least 13 times that of prior art Form I. Form CSIII of the present disclosure exhibits higher solubility in common organic solvents, which is beneficial for production and preparation. It also reduces the solvent volume required in the production process, making it more energy-efficient and environmentally friendly.
(4) Form CSIII of the present disclosure has no residual solvent. Residual solvents can affect drug safety, quality and stability, potentially leading to crystalline transformation or impurity formation during drug's production and storage, which can affect drug bioavailability and toxicity. The absence of residual solvents in Form CSIII effectively addresses issues related to low drug stability, poor efficacy, and high toxicity caused by low drug purity or high solvent residues.
(5) Form CSIII of the present disclosure has good purification effect. The purity is significantly increased after the starting material is converted into Form CSIII. In a specific embodiment of the present disclosure, the purity of the starting material used is 99.14%. The purity of Form CSIII made from the starting material is 99.86%. The purity is increased over 0.72%. Chemical purity is of great significance for ensuring drug efficacy, safety and preventing the occurrence of adverse effects. Drug regulations have strict requirements on impurity content. As Form CSIII of the present disclosure has good purification ability and is excellent in removing impurities in the crystallization process. Thus drug substances with high purity can be obtained through crystallization, which effectively overcome the disadvantages of poor stability, poor efficacy and high toxicity caused by the low purity drug substances.
(6) Form CSIII of the present disclosure has good physical stability under mechanical force. The crystalline state of Form CSIII doesn't change after tableting, grinding and formulation process. Grinding and pulverization are often required in the drug manufacturing process. Good physical stability of the drug substance can reduce the risk of crystallinity decrease and crystal transformation during the drug manufacturing process. In addition, Form CSIII has good physical stability under different pressures, which is beneficial to keep crystalline form unchanged during tableting process.
(7) Form CSIII of the present disclosure has good photostability. When Form CSIII is exposed to light under certain conditions for at least two weeks, it remains unchanged, and its purity remains relatively stable. Light can accelerate drug oxidation, cause photodegradation, and lead to color changes and precipitation, which not only reduces the drug's potency but also severely affects its quality and can even increase toxicity. Therefore, the stability of drug substance under light is crucial for drug. Form CSIII has good photostability, which helps prevent impacts on drug quality due to crystal transformation or purity degradation during storage.
(8) From CSIII drug substance and drug product of the present disclosure have good stability. Crystalline state of Form CSIII drug substance doesn't change for at least 9 months when stored under the condition of 25 °C/60%RH. The chemical purity remains substantially unchanged during storage. After Form CSIII is mixed with the excipients to form a drug product and stored under the condition of 25 °C/60%RH, crystalline state of Form CSIII drug product doesn't change for at least one month. These results show that From CSIII drug substance and drug product of the present disclosure have good stability under long term condition, which is beneficial to the drug storage. Meanwhile, crystalline state of Form CSIII drug substance doesn't change for at least 3 months when stored under the condition of 40 °C/75%RH. The crystalline state of Form CSIII drug substance doesn't change for at least 2 months when stored under the condition of 60 °C/75%RH. The chemical purity is above 99% and remains substantially unchanged during storage. These results show that Form CSIII drug substance has good stability under accelerated and stress conditions. Drug substance and drug product would go through high temperature and high humidity conditions caused by different season, regional climate and environment during storage, transportation, and manufacturing processes. Therefore, good stability under accelerated and stress conditions is of great importance to the drug development. Form CSIII drug substance and drug product have good stability under stress condition, which is beneficial to avoid the impact on drug quality due to crystal transformation or decrease in purity during drug storage.
   Good physical and chemical stability of drug substance ensure that no crystal transformation or impurities is generated during production and storage. Form CSIII has good physical and chemical stability, ensuring consistent and controllable quality of the drug substance and drug product, minimizing quality change, bioavailability change and toxicity due to crystal transformation or impurity generation.
(9) Form CSIII drug product of the present disclosure has good in vitro dissolution. In pH 6.8 PBS, the dissolution of Form CSIII drug product at 15 minutes is up to 97.5%. Drug dissolution is a prerequisite for drug absorption. Drugs with different crystalline forms may lead different in vivo dissolution, which ultimately leads to different clinical efficacy. According to BCS guidelines, in vitro dissolution may be relevant to the prediction of in vivo performance of drug products. Good in vitro dissolution of Form CSIII drug products provided by the present disclosure may leads to higher in vivo absorption, and better in vivo exposure, thereby improving drug's bioavailability and efficacy. Higher intrinsic dissolution rate of Form CSIII drug substance is beneficial for the drug to achieve peak concentration in plasma quickly after administration, thus ensuring rapid drug action.

Form CSIV of the present disclosure has the following beneficial effects:
(1) Compared with prior art Form I, Form CSIV of the present disclosure has better compressibility. Failure in hardness/friability test and tablet crack issue can be avoided due to better compressibility, making the preparation process more reliable, improving product appearance, promoting product quality and production efficiency.
(2) Compared with prior art Form I, Form CSIV of the present disclosure has better flowability. Flowability evaluation results indicate that the flowability of Form CSIV is remarkably better than that of prior art forms. Better flowability can prevent clogging of production equipment and increase manufacturing efficiency. Better flowability of Form CSIV ensures the content uniformity of the drug product, reduces the weight variation of the drug product and improves product quality.
(3) Form CSIV of the present disclosure has good physical stability under mechanical force. The crystalline state of Form CSIV doesn't change after grinding. Grinding and pulverization are often required in the drug manufacturing process. Good physical stability of the drug substance can reduce the risk of crystallinity decrease and crystal transformation during the drug manufacturing process.
(4) Form CSIV of the present disclosure has good photostability. When Form CSIV is exposed to light under certain conditions for at least two weeks, it remains unchanged, and its purity remains relatively stable. Light can accelerate drug oxidation, cause photodegradation, and lead to color changes and precipitation, which not only reduces the drug's potency but also severely affects its quality and can even increase toxicity. Therefore, the stability of drug substance under light is crucial for drug. Form CSIV has good photostability, which helps prevent impacts on drug quality due to crystal transformation or purity degradation during storage.
(5) From CSIV drug substance and drug product of the present disclosure have good stability. Crystalline state of Form CSIV drug substance doesn't change for at least 6 months when stored under the condition of 25 °C/60%RH. The chemical purity remains substantially unchanged during storage. These results show that From CSIV drug substance has good stability under long term condition, which is beneficial to the drug storage.

Meanwhile, crystalline state of Form CSIV drug substance doesn't change for at least 6 months when stored under the condition of 40 °C/75%RH. The crystalline state of Form CSIV drug substance doesn't change for at least 2 weeks when stored under the condition of 60 °C/75%RH. The chemical purity is above 99.9% and remains substantially unchanged during storage. These results show that Form CSIV drug substance has good stability under accelerated and stress conditions. Drug substance and drug product would go through high temperature and high humidity conditions caused by different season, regional climate and environment during storage, transportation, and manufacturing processes. Therefore, good stability under accelerated and stress conditions is of great importance to the drug development. Form CSIV drug substance has good stability under stress condition, which is beneficial to avoid the impact on drug quality due to crystal transformation or decrease in purity during drug storage.

Good physical and chemical stability of drug substance ensure that no crystal transformation or impurities is generated during production and storage. Form CSIV has good physical and chemical stability, ensuring consistent and controllable quality of the drug substance and drug product, minimizing quality change, bioavailability change and toxicity due to crystal transformation or impurity generation.

### BRIEF DESCRIPTION OF THE DRAWINGS

Figure 1 shows an XRPD pattern of Form CSII
Figure 2 shows a TGA curve of Form CSII
Figure 3 shows a DSC curve of Form CSII
Figure 4 shows an XRPD pattern of Form CSII
Figure 5 shows a TGA curve of Form CSIII
Figure 6 shows a DSC curve of Form CSIII
Figure 7 shows an XRPD pattern of Form CSIII
Figure 8 shows an XRPD pattern of Form CSIV
Figure 9 shows a TGA curve of Form CSIV
Figure 10 shows an XRPD pattern of Form CSIV
Figure 11 shows a DSC curve of Form CSIV
Figure 12 shows an XRPD pattern overlay of Form CSII before and after storage under different conditions (from top to bottom: initial, 25°C/60%RH sealed with desiccants for 9 months, 25°C/60%RH open for 9 months, 40 °C/75%RH sealed with desiccants for 6 months, 60 °C/75%RH sealed with desiccants for one month)
Figure 13 shows an XRPD pattern overlay of Form CSII before and after tableting (from top to bottom: 0 kN pressure, 5 kN pressure, 10 kN pressure, 20 kN pressure).
Figure 14 shows an XRPD pattern overlay of Form CSII before and after ball milling (from top to bottom: before ball milling, after ball milling).
Figure 15 shows an XRPD pattern overlay of Form CSII before and after formulation process (from top to bottom: excipients, Form CSII drug product, Form CSII)
Figure 16 shows a dissolution curve of Form CSII
Figure 17 shows an XRPD pattern overlay of Form CSII product before and after storage under 25 °C/60%RH sealed with 1 g of desiccant (from top to bottom: before storage, after one month storage)
Figure 18 shows an XRPD pattern overlay of Form CSIII before and after storage under different conditions (Form top to bottom: initial, 25°C/60%RH sealed with desiccants for 9 months, 25°C/60%RH open for 9 months, 40 °C/75%RH sealed with desiccants for 3 months, 60 °C/75%RH sealed with desiccants for 2 months)
Figure 19 shows an XRPD pattern overlay of Form CSIII before and after formulation process (from top to bottom: excipients, Form CSIII drug product, Form CSIII)
Figure 20 shows a dissolution curve of Form CSIII
Figure 21 shows an XRPD pattern overlay of Form CSIII product before and after storage under 25 °C/60%RH sealed with 1 g of desiccant (from top to bottom: before storage, after one month storage)
Figure 22 shows an XRPD pattern overlay of Form CSIV before and after storage under different conditions (Form top to bottom: initial, 25°C/60%RH sealed with desiccants for 6 months, 40 °C/75%RH sealed with desiccants for 6 months, 60 °C/75%RH sealed with desiccants for two weeks)
Figure 23 shows an XRPD pattern overlay of Form CSIV before and after ball milling (from top to bottom: before ball milling, after ball milling).

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.
The abbreviations used in the present disclosure are explained as follows:
RH: Relative humidity
XRPD: X-ray Powder Diffraction
TGA: Thermo Gravimetric Analysis
DSC: Differential Scanning Calorimetry
DVS: Dynamic Vapor Sorption
¹H NMR: Proton Nuclear Magnetic Resonance
HPLC: High Performance Liquid Chromatography
HDPE: High Density Polyethylene
PBS: Phosphate Buffer Solution

### Instruments and methods used for data collection:

XRPD patterns in the present disclosure were acquired by an X-ray powder diffractometer. The parameters of the XRPD method of the present disclosure are as follows:
X-Ray source: Cu, Kα
Kα1 (Å): 1.54060; Kα2 (Å): 1.54439
Kα2/Kα1 intensity ratio: 0.50

DSC data in the present disclosure were acquired by a TA Q2000. The parameters of the DSC method of the present disclosure are as follows:
Heating rate: 10 °C/min
Purge gas: N₂

TGA data in the present disclosure were acquired by a TA Q500. The parameters of the TGA method of the present disclosure are as follows:
Heating rate: 10 °C/ min
Purge gas: N₂

DVS data in the present disclosure were measured via an SMS (Surface Measurement Systems Ltd.) intrinsic DVS instrument. The instrument control software is DVS-Intrinsic control software. Typical Parameters for DVS test are as follows:
Temperature: 25 °C
Gas and flow rate: N₂, 200 mL/min
RH range: 0% RH to 95% RH

¹H NMR data were collected from a Bruker Avance II DMX 400M HZ NMR spectrometer. 1-5 mg of sample was weighed and dissolved with 0.5 mL of deuterated dimethyl sulfoxide to obtain a solution with a concentration of 2-10 mg/mL.

The parameters for related substance detection in the present disclosure are shown in Table 1.

**Table 1**

| HPLC | Agilent 1260 | |
|---|---|---|
| Mobile phase | A: 0.1% phosphoric acid aqueous solution (pH2.5, triethylamine) | |
| | B: Acetonitrile | |
| Gradient | Time (min) | %B |
| | 0.0 | 20 |
| | 0.5 | 20 |
| | 22.0 | 90 |
| | 25.0 | 90 |
| | 25.1 | 20 |
| | 35.0 | 20 |
| Run time | 35.0 min | |
| Post time | 0.0 min | |
| Flow rate | 1.0 mL/min | |
| Injection volume | 5 µL | |
| Detector wavelength | 210 nm | |
| Column temperature | 40 °C | |
| Sample temperature | Room temperature | |
| Diluent | Acetonitrile: H₂O = 50: 50 (v/v) | |

The parameters for content detection in the present disclosure are shown in Table 2.

**Table 1**

| HPLC | Agilent 1260 | |
|---|---|---|
| Column | Waters Xbridge C18, 4.6 mm × 150 mm, 3.5 µm | |
| Mobile phase | A: 0.1% phosphoric acid aqueous solution | |
| | B: Acetonitrile | |
| Gradient | Time (min) | %B |
| | 0.0 | 20 |
| | 0.5 | 20 |
| | 6.0 | 80 |
| | 8.0 | 80 |
| | 8.1 | 20 |
| | 10.0 | 20 |
| Run time | 10.0 min | |
| Post time | 0.0 min | |
| Flow rate | 1.0 mL/min | |
| Injection volume | 10 µL | |
| Detector wavelength | 210 nm | |
| Column temperature | 40 °C | |
| Sample temperature | Room temperature | |

In the present disclosure, said "co-crystal" is crystalline materials composed of two or more different molecules (one of which is the API) in a defined stoichiometric ratio within the same crystal lattice that are associated by nonionic and noncovalent bonds. Said "anhydrate" refers to a solid substance that does not contain crystalline water or solvents.

Said "stirring" is accomplished by using a conventional method in the field such as magnetic stirring or mechanical stirring and the stirring speed is 50 to 1800 r/min. Preferably the magnetic stirring speed is 300 to 900 r/min, and mechanical stirring speed is 100 to 300 r/min.

Said "separation" is accomplished by using a conventional method in the field such as centrifugation or filtration. The operation of "centrifugation" is as follows: the sample to be separated is placed into the centrifuge tube, and then centrifuged at a rate of 10000 r/min until the solid all sink to the bottom of the tube.

Said "malic acid" can be DL malic acid, L-malic acid, or D-malic acid.

Said "tartaric acid" can be DL tartaric acid, L-tartaric acid, or D-tartaric acid.

Said "room temperature" is not a specific temperature, but a temperature range of 10-30 °C.

Said "characteristic peak" refers to a representative diffraction peak used to distinguish crystals, which usually can have a deviation of ±0.2° using CuKα radiation.

In the present disclosure, "crystal" or "crystalline form" refers to the crystal or the crystalline form being identified by the X-ray diffraction pattern shown herein. Those skilled in the art are able to understand that the X-ray powder diffraction pattern depend on the instrument conditions, the sample preparation and the purity of samples. The relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSII, Form CSIII and Form CSIV of the present disclosure are pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature.

According to the present disclosure, Compound I used as raw materials includes, but is not limited to solid (crystalline or amorphous), oil, liquid form or solution. Preferably, Compound I used as the raw material is a solid.

Raw materials of Compound I used in the following examples were prepared by known methods in the prior art, for example, the method disclosed in WO2017013498A1.

### Example 1 Preparation of Form CSII

22.8 mg of Compound I hydrochloride and 10.8 mg of L-tartaric acid were weighed into a centrifuge tube, and 10 µL of methyl isobutyl ketone was added. After ball milling at 1000 rpm for 1 hour, another 0.4 mL of methyl isobutyl ketone was added. The mixture was suspended and stirred at room temperature for 9 days. Then the solid was separated by centrifugation and vacuum dried at 50 °C for 15 minutes. Subsequently, about 0.6 mL of 2-methyltetrahydrofuran was added, then the solid was separated, and vacuum dried at 30 °C for 25 minutes to obtain a crystalline solid.

The obtained crystalline solid was confirmed as Form CSII after testing. The XRPD pattern is shown in Figure 1, and the XRPD data are listed in Table 3.

The TGA curve of Form CSII is shown in Figure 2, which shows Form CSII has an approximately weight loss of 0.2% when heated to 130 °C.

The DSC curve of Form CSII is shown in Figure 3, which shows there is one endothermic peak. The endothermic peak at around 163 °C (onset temperature) corresponds to a melting process.

¹H NMR data of Form CSII are: ¹H NMR (400 MHz, DMSO-d6) δ 7.64 - 7.58 (m, 2H), 7.43 - 7.37 (m, 2H), 7.37 - 7.27 (m, 4H), 7.27 - 7.17 (m, 2H), 4.31 (s, 1H), 4.16 (q, J = 7.8 Hz, 1H), 3.76 (td, J = 8.8, 4.8 Hz, 1H), 3.60 - 3.51 (m, 1H), 2.86 - 2.68 (m, 8H), 2.23 - 2.12 (m, 1H), 2.03 - 1.86 (m, 1H). The ¹H NMR result shows that there is almost no residual solvent in Form CSII, and the molar ratio of Compound I to L-tartaric acid in the Form CSII is 1:0.5. The NMR signal of the active hydrogen of HCl in the Compound I hydrochloride is not detected, and the NMR signal at the chemical shift of 4.31 ppm is the signal of L-tartaric acid.

**Table 3**

| 2θ (°) | d-spacing (Å) | Intensity (%) |
|---|---|---|
| 5.09 | 17.36 | 21.13 |
| 6.83 | 12.94 | 15.27 |
| 8.62 | 10.26 | 43.27 |
| 10.19 | 8.68 | 13.60 |
| 11.19 | 7.91 | 14.78 |
| 11.94 | 7.42 | 14.23 |
| 12.42 | 7.13 | 5.34 |
| 12.77 | 6.93 | 44.53 |
| 13.05 | 6.78 | 25.16 |
| 13.46 | 6.58 | 20.14 |
| 14.72 | 6.02 | 36.76 |
| 15.32 | 5.78 | 31.69 |
| 15.55 | 5.70 | 31.60 |
| 16.48 | 5.38 | 60.42 |
| 16.63 | 5.33 | 42.46 |
| 16.90 | 5.25 | 22.18 |
| 17.10 | 5.19 | 42.87 |
| 17.29 | 5.13 | 33.76 |
| 17.51 | 5.07 | 46.73 |
| 18.04 | 4.92 | 16.31 |
| 19.29 | 4.60 | 12.02 |
| 19.75 | 4.49 | 30.11 |
| 19.86 | 4.47 | 25.59 |
| 20.46 | 4.34 | 100.00 |
| 20.76 | 4.28 | 12.55 |
| 21.10 | 4.21 | 44.57 |
| 21.40 | 4.15 | 10.19 |
| 21.76 | 4.08 | 46.92 |
| 22.30 | 3.99 | 18.04 |
| 22.60 | 3.94 | 8.92 |
| 24.19 | 3.68 | 10.89 |
| 24.41 | 3.65 | 15.98 |
| 24.86 | 3.58 | 15.85 |
| 25.30 | 3.52 | 13.69 |
| 25.66 | 3.47 | 24.85 |
| 26.95 | 3.31 | 15.00 |
| 27.55 | 3.24 | 32.38 |
| 28.97 | 3.08 | 11.68 |
| 29.21 | 3.06 | 12.47 |
| 29.61 | 3.02 | 5.98 |
| 30.53 | 2.93 | 5.87 |
| 30.94 | 2.89 | 11.39 |
| 31.38 | 2.85 | 5.40 |
| 34.61 | 2.59 | 10.87 |
| 37.46 | 2.40 | 5.01 |
| 38.43 | 2.34 | 5.49 |

### Example 2 Preparation of Form CSII

16.8 mg of Compound I hydrochloride, 1.8 mg of Form CSII obtained from Example 1 (as seed) and 12.1 mg of L-tartaric acid were weighed into a vial, and 0.4 mL of 2-methyltetrahydrofuran was added. After the mixture was suspended and stirred at room temperature for 2 days, 4.0 mg of L-tartaric acid was added, and then the suspension was stirred at room temperature for another 1 day. Another 0.6 mg of L-tartaric acid was added, and the suspension was stirred at room temperature for another 3 days. The solid was separated, and vacuum dried at room temperature for 35 minutes, then the crystalline solid was obtained.

The obtained crystalline solid was confirmed as Form CSII after testing. The XRPD pattern is shown in Figure 4, and the XRPD data are listed in Table 4.

**Table 4**

| 2θ (°) | d-spacing (Å) | Intensity (%) |
|---|---|---|
| 5.10 | 17.32 | 16.15 |
| 6.84 | 12.92 | 10.36 |
| 8.63 | 10.25 | 26.02 |
| 10.20 | 8.67 | 10.68 |
| 11.19 | 7.91 | 9.63 |
| 11.92 | 7.42 | 10.28 |
| 12.77 | 6.93 | 25.65 |
| 13.05 | 6.79 | 14.92 |
| 13.46 | 6.58 | 14.07 |
| 14.75 | 6.01 | 18.83 |
| 15.32 | 5.79 | 31.31 |
| 15.55 | 5.70 | 19.84 |
| 16.01 | 5.53 | 6.07 |
| 16.48 | 5.38 | 30.53 |
| 16.63 | 5.33 | 26.06 |
| 16.91 | 5.24 | 15.66 |
| 17.12 | 5.18 | 21.82 |
| 17.31 | 5.12 | 17.47 |
| 17.49 | 5.07 | 28.64 |
| 18.04 | 4.92 | 8.77 |
| 19.28 | 4.60 | 6.24 |
| 19.76 | 4.49 | 22.07 |
| 20.17 | 4.40 | 10.90 |
| 20.46 | 4.34 | 100.00 |
| 20.76 | 4.28 | 14.74 |
| 21.11 | 4.21 | 33.14 |
| 21.77 | 4.08 | 29.86 |
| 22.29 | 3.99 | 14.01 |
| 22.61 | 3.93 | 6.79 |
| 24.18 | 3.68 | 6.63 |
| 24.42 | 3.65 | 11.58 |
| 24.88 | 3.58 | 14.17 |
| 25.32 | 3.52 | 11.10 |
| 25.66 | 3.47 | 26.87 |
| 26.93 | 3.31 | 7.79 |
| 27.59 | 3.23 | 18.94 |
| 29.22 | 3.06 | 11.05 |
| 30.70 | 2.91 | 5.48 |
| 30.92 | 2.89 | 8.78 |

### Example 3 Preparation of Form CSIII

22.1 mg of Compound I hydrochloride and 20.1 mg of citric acid were weighed into a centrifuge tube, and 10 µL of methyl isobutyl ketone was added. After ball milling at 1000 rpm for 1 hour, 400 µL of methyl isobutyl ketone was added, then the mixture was suspended and stirred at room temperature for 6 days. The solid was separated by centrifugation and vacuum dried at room temperature for about 2.5 hours, the crystalline solid was obtained.

The obtained crystalline solid was confirmed as Form CSIII after testing. The XRPD data are listed in Table 5.

The TGA curve of Form CSIII is shown in Figure 5, which shows Form CSIII has an approximately weight loss of 0.1% when heated to 100 °C.

The DSC curve of Form CSIII is shown in Figure 6, which shows there are two endothermic peaks. The first endothermic peak is around 104 °C (onset temperature) and the second peak is around 186 °C (peak temperature).

¹H NMR data of Form CSIII are: ¹H NMR (400 MHz, DMSO-d6) δ 7.66 - 7.56 (m, 2H), 7.43 - 7.38 (m, 2H), 7.37- 7.27 (m, 4H), 7.26 - 7.17 (m, 2H), 4.16 (q, J = 7.8 Hz, 1H), 3.76 (td, J = 8.8, 4.8 Hz, 1H), 3.62 - 3.51 (m, 1H), 2.83 - 2.60 (m, 12H), 2.28 - 2.16 (m, 1H), 1.98 - 1.87 (m, 1H). The ¹H NMR result shows that there is almost no residual solvent in Form CSIII, and the molar ratio of Compound I to citric acid in the Form CSIII is 1:1. The NMR signals of four hydrogen atoms on the alkyl chain of citric acid are included in the signals at 2.83-2.60 ppm, and the NMR signals of the active hydrogen atoms of citric acid and HCl in the Compound I hydrochloride are not detected.

**Table 5**

| 2θ (°) | d-spacing (Å) | Intensity (%) |
|---|---|---|
| 4.23 | 20.87 | 32.59 |
| 8.57 | 10.32 | 2.81 |
| 11.12 | 7.96 | 25.19 |
| 12.94 | 6.84 | 19.41 |
| 15.14 | 5.85 | 8.29 |
| 16.56 | 5.35 | 100.00 |
| 20.17 | 4.40 | 66.55 |
| 21.07 | 4.22 | 46.19 |
| 21.68 | 4.10 | 4.92 |
| 22.98 | 3.87 | 4.76 |
| 23.88 | 3.73 | 17.71 |
| 24.45 | 3.64 | 8.61 |
| 26.14 | 3.41 | 5.86 |
| 26.94 | 3.31 | 9.18 |
| 31.44 | 2.85 | 6.19 |
| 33.65 | 2.66 | 6.13 |
| 34.87 | 2.57 | 4.38 |
| 36.37 | 2.47 | 2.05 |

### Example 4 Preparation of Form CSIII

19.9 mg of Compound I hydrochloride and 24.3 mg of citric acid were weighed into a vial, and 200 µL of methyl isobutyl ketone was added. The mixture was suspended and stirred at room temperature for 5 days. With a small amount of seed of Form CSIII obtained from Example 3 added, the mixture was suspended and stirred at room temperature for another 1 day. The suspension was stood at room temperature for 18 days, then the solid was separated. Subsequently, 1 mL of methyl isobutyl ketone was added, and then the crystalline solid was separated.

The obtained crystalline solid was confirmed as Form CSIII after testing. The XRPD data are listed in Table 6. 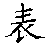 2

**Table 6**

| 2θ (°) | d-spacing (Å) | Intensity (%) |
|---|---|---|
| 4.26 | 20.75 | 35.22 |
| 8.58 | 10.31 | 4.30 |
| 11.11 | 7.96 | 37.72 |
| 12.93 | 6.85 | 24.53 |
| 15.17 | 5.84 | 4.83 |
| 16.56 | 5.35 | 100.00 |
| 20.17 | 4.40 | 71.73 |
| 21.07 | 4.22 | 63.86 |
| 21.72 | 4.09 | 7.49 |
| 23.89 | 3.72 | 15.51 |
| 24.47 | 3.64 | 8.74 |
| 25.21 | 3.53 | 3.80 |
| 26.13 | 3.41 | 6.67 |
| 26.97 | 3.31 | 9.20 |
| 30.22 | 2.96 | 1.87 |
| 31.48 | 2.84 | 9.79 |
| 32.38 | 2.76 | 2.40 |
| 33.59 | 2.67 | 10.53 |
| 34.92 | 2.57 | 5.98 |
| 36.34 | 2.47 | 3.34 |

### Example 5 Preparation of Form CSIII

19.6 mg of Compound I hydrochloride and 15.1 mg of citric acid were weighed into a vial, and 0.4 mL of methyl isobutyl ketone was added. The mixture was suspended and stirred at 50 °C for 3 days, then 0.6 mL of methyl isobutyl ketone saturated with citric acid was added. The suspension was stirred at room temperature for another 15 days. The solid was separated by centrifugation and vacuum dried at 30 °C for 6 hours, then the crystalline solid was obtained.

The obtained crystalline solid was confirmed as Form CSIII after testing. The XRPD pattern is shown in Figure 7, and the XRPD data are listed in Table 7.

**Table 7**

| 2θ (°) | d-spacing (Å) | Intensity (%) |
|---|---|---|
| 4.33 | 20.43 | 60.16 |
| 8.67 | 10.20 | 6.78 |
| 11.17 | 7.92 | 29.38 |
| 13.02 | 6.80 | 40.11 |
| 14.31 | 6.19 | 8.18 |
| 15.18 | 5.84 | 45.07 |
| 16.62 | 5.33 | 100.00 |
| 17.13 | 5.18 | 5.31 |
| 17.66 | 5.02 | 18.41 |
| 18.21 | 4.87 | 21.73 |
| 20.26 | 4.38 | 97.96 |
| 21.14 | 4.20 | 34.17 |
| 21.79 | 4.08 | 13.10 |
| 23.01 | 3.87 | 19.70 |
| 23.94 | 3.72 | 14.63 |
| 24.13 | 3.69 | 28.88 |
| 24.52 | 3.63 | 10.58 |
| 25.24 | 3.53 | 5.90 |
| 26.20 | 3.40 | 5.66 |
| 26.40 | 3.38 | 11.97 |
| 27.14 | 3.29 | 26.21 |
| 27.62 | 3.23 | 6.47 |
| 28.23 | 3.16 | 9.68 |
| 28.86 | 3.09 | 5.90 |
| 29.28 | 3.05 | 9.36 |
| 30.18 | 2.96 | 7.79 |
| 31.40 | 2.85 | 9.41 |
| 32.44 | 2.76 | 5.37 |
| 33.10 | 2.71 | 5.75 |
| 33.63 | 2.66 | 11.10 |
| 34.89 | 2.57 | 6.55 |
| 35.21 | 2.55 | 9.46 |
| 36.34 | 2.47 | 5.37 |
| 39.52 | 2.28 | 7.85 |

### Example 6 Preparation of Form CSIV

13.9 mg of Compound I hydrochloride and 6.1 mg of DL-malic acid were weighed into a vial, and 0.2 mL of methyl isobutyl ketone was added. The mixture was suspended and stirred at 50 °C for 7 days. The solid was separated by centrifugation and vacuum dried at room temperature for about 3 hours, then the crystalline solid was obtained.

The obtained crystalline solid was confirmed as Form CSIV after testing. The XRPD pattern is shown in Figure 8, and the XRPD data are listed in Table 8.

The TGA curve of Form CSIV is shown in Figure 9, which shows Form CSIV has an approximately weight loss of 0.3% when heated to 100 °C.

¹H NMR data of Form CSIV are: ¹H NMR (400 MHz, DMSO-d6) δ 9.79 (s, 1H), 7.67 - 7.56 (m, 2H), 7.45 - 7.38 (m, 2H), 7.37 - 7.28 (m, 4H), 7.26 - 7.17 (m, 2H), 4.26 (dd, J = 7.8, 4.8 Hz, 0.5H), 4.16 (q, J = 7.8 Hz, 1H), 3.76 (td, J = 8.8, 4.7 Hz, 1H), 3.62 - 3.52 (m, 1H), 2.92 - 2.66 (m, 8H), 2.65 - 2.57 (m, 0.5H), 2.47 - 2.39 (m, 0.5H), 2.28 - 2.16 (m, 1H), 2.03 - 1.86 (m, 1H). The ¹H NMR result shows that there is almost no residual solvent in Form CSIV, and the molar ratio of Compound I to DL-malic acid in Form CSIV is 1:0.5. The NMR signals of the DL-malic acid are at 4.31 ppm, 2.65-2.57 ppm and 2.47-2.39 ppm, and NMR signals of active hydrogen atoms of hydroxyl and carboxyl groups of DL-malic acid are not detected.

**Table 8**

| 2θ (°) | d-spacing (Å) | Intensity (%) |
|---|---|---|
| 5.08 | 17.41 | 7.45 |
| 6.76 | 13.08 | 11.26 |
| 8.56 | 10.33 | 13.62 |
| 10.18 | 8.69 | 16.07 |
| 11.14 | 7.94 | 14.85 |
| 11.95 | 7.41 | 10.77 |
| 12.72 | 6.96 | 19.86 |
| 13.07 | 6.77 | 13.11 |
| 13.39 | 6.61 | 17.81 |
| 14.63 | 6.05 | 16.14 |
| 15.06 | 5.88 | 8.58 |
| 15.31 | 5.79 | 31.79 |
| 15.60 | 5.68 | 23.29 |
| 16.53 | 5.36 | 43.62 |
| 16.64 | 5.33 | 36.68 |
| 16.83 | 5.27 | 13.40 |
| 17.09 | 5.19 | 30.69 |
| 17.22 | 5.15 | 17.83 |
| 17.53 | 5.06 | 26.25 |
| 17.94 | 4.94 | 8.07 |
| 19.28 | 4.60 | 9.67 |
| 19.60 | 4.53 | 11.02 |
| 19.78 | 4.49 | 32.54 |
| 19.93 | 4.46 | 17.33 |
| 20.48 | 4.34 | 100.00 |
| 20.72 | 4.29 | 16.94 |
| 20.98 | 4.23 | 22.84 |
| 21.76 | 4.08 | 38.92 |
| 22.19 | 4.01 | 17.95 |
| 22.51 | 3.95 | 7.64 |
| 24.07 | 3.70 | 5.87 |
| 24.25 | 3.67 | 8.67 |
| 24.78 | 3.59 | 15.45 |
| 25.20 | 3.53 | 16.60 |
| 25.50 | 3.49 | 10.29 |
| 25.68 | 3.47 | 26.56 |
| 26.82 | 3.32 | 10.72 |
| 27.39 | 3.26 | 12.09 |
| 27.70 | 3.22 | 5.08 |
| 28.87 | 3.09 | 10.61 |
| 29.08 | 3.07 | 7.99 |
| 29.56 | 3.02 | 4.35 |
| 30.37 | 2.94 | 7.08 |
| 30.74 | 2.91 | 7.45 |

### Example 7 Preparation of Form CSIV

10.1 mg of Compound I hydrochloride and 4.5 mg of L-malic acid were weighed into a vial, and 0.2 mL of methyl isobutyl ketone was added. The mixture was suspended and stirred at room temperature for 1 day, then 4.8 mg of L-malic acid was added. The suspension was stirred at room temperature for another 7 days. The solid was separated, and vacuum dried at 30 °C for about 2 hours, then the crystalline solid was obtained.

The obtained crystalline solid was confirmed as Form CSIV after testing. The XRPD pattern is shown in Figure 10, and the XRPD data are listed in Table 9.

**Table 9**

| 2θ (°) | d-spacing (Å) | Intensity (%) |
|---|---|---|
| 5.04 | 17.53 | 17.71 |
| 6.75 | 13.10 | 15.36 |
| 8.55 | 10.34 | 15.79 |
| 10.16 | 8.71 | 15.88 |
| 11.13 | 7.95 | 8.16 |
| 11.95 | 7.41 | 6.54 |
| 12.70 | 6.97 | 16.13 |
| 13.06 | 6.78 | 8.40 |
| 13.38 | 6.62 | 10.31 |
| 14.41 | 6.15 | 4.22 |
| 14.63 | 6.05 | 14.11 |
| 15.05 | 5.89 | 5.46 |
| 15.30 | 5.79 | 37.13 |
| 15.58 | 5.69 | 14.45 |
| 16.56 | 5.35 | 30.31 |
| 16.81 | 5.27 | 8.93 |
| 17.09 | 5.19 | 21.30 |
| 17.24 | 5.14 | 15.37 |
| 17.52 | 5.06 | 17.20 |
| 17.92 | 4.95 | 9.94 |
| 19.27 | 4.61 | 6.38 |
| 19.60 | 4.53 | 8.76 |
| 19.77 | 4.49 | 21.48 |
| 19.92 | 4.46 | 9.39 |
| 20.47 | 4.34 | 100.00 |
| 20.72 | 4.29 | 11.88 |
| 20.96 | 4.24 | 16.91 |
| 21.33 | 4.17 | 5.79 |
| 21.76 | 4.08 | 26.67 |
| 22.18 | 4.01 | 11.27 |
| 22.45 | 3.96 | 5.61 |
| 22.55 | 3.94 | 5.78 |
| 24.05 | 3.70 | 5.17 |
| 24.25 | 3.67 | 6.86 |
| 24.76 | 3.60 | 10.67 |
| 25.16 | 3.54 | 10.53 |
| 25.48 | 3.50 | 7.80 |
| 25.67 | 3.47 | 26.04 |
| 26.82 | 3.32 | 7.54 |
| 27.38 | 3.26 | 11.16 |
| 27.70 | 3.22 | 5.73 |
| 28.84 | 3.10 | 7.23 |
| 29.05 | 3.07 | 4.22 |
| 30.73 | 2.91 | 5.10 |
| 30.92 | 2.89 | 6.19 |

### Example 8 DSC curve of Form CSIV

The DSC curve of Form CSIV is shown in Figure 11, which shows there are three endothermic peaks. The first endothermic peak is at 148 °C (onset temperature), the second endothermic peak is at 177 °C (peak temperature) and the third endothermic peak is at 220 °C (peak temperature).

### Example 9 Hygroscopicity of Form CSII

Dynamic vapor sorption (DVS) analyzer was applied to evaluate the hygroscopicity of Form CSII and Form I in the prior art with about 10 mg of sample, respectively. The mass change at each relative humidity were recorded in a cycle of 0%RH-95%RH-0%RH. The results are listed in Table 10. The results show that the hygroscopic mass increase of Form I in the prior art under 70%RH condition is 2.3 times that of Form CSII, and the hygroscopicity of Form CSII is better than that of Form I in the prior art.

**Table 10**

| Solid form | Mass increase under 70%RH |
|---|---|
| Form CSII | 0.24% |
| Form I in the prior art | 0.54% |

### Example 10 Compressibility of CSII

The samples were pressed by the ENERPAC single punch manual tablet press. 80 mg of Form CSII and Form I in the prior art were added into dies of a Φ6mm round tooling, and compressed into tablets under a pressure of 3 kN, respectively. The elastic recovery was completed after storage at room temperature for 24 hours, then the diameter (D) and thickness (L) of the tablet was measured by a caliper, and the hardness (H) was tested with a tablet hardness tester. Tensile strength of the powder was calculated with the following formula: T=2H/πDL. Under a certain force, the greater the tensile strength, the better the compressibility. The results are listed in Table 11. The results indicate Form CSII has better compressibility compared with Form I in the prior art.

**Table 11**

| Solid form | Tensile strength (MPa) |
|---|---|
| Form I in the prior art | 0.552 |
| Form CSII | 0.705 |

### Example 11 Solubility of Form CSII

About 3-5 mg of Form CSII or Form I in the prior art was weighed into each vial respectively, and 0.05 mL of corresponding solvent was added step-wise till the solid was totally dissolved. The weight of solid (m), the undissolved volume (v₁) and the dissolved volume (v₂) were recorded respectively. According to the formula: m/v₂<S<m/v₁, which m needs to be converted from the weighed mass to the corresponding mass of Compound I, the solubility (S) results are calculated and shown in Table 12.

The results show that in common alcohol, ketone and ether solvents, the solubility of Form CSII is higher than that of Form I in the prior art, especially in n-propanol, the solubility of Form CSII is at least 2-4 times that of Form I in the prior art.

**Table 12**

| Solvent | Solubility of Form I in the prior art (mg/mL) | Solubility of Form CSII (mg/mL) |
|---|---|---|
| n-Propanol | 6.3<S<12.5 | S>26.0 |
| Acetone | 1.5<S<2.2 | 4.6<S<5.8 |
| Tetrahydrofuran | S<1.5 | S<1.8 |

### Example 12 Light stability of Form CSII

An appropriate amount of Form CSII of the present disclosure was placed in a light stability chamber with the following setting values: light: 8 k Lux, UV: 1.5 w/m². The chemical purity and crystalline form were tested by HPLC and XRPD respectively, and the results are shown in Table 13. The results show that Form CSII has good light stability.

**Table 13**

| Initial form | Initial purity | Time | Solid form | Purity |
|---|---|---|---|---|
| Form CSII | 99.97% | 2 weeks | Form CSII | 99.97% |

### Example 13 Stability of Form CSII

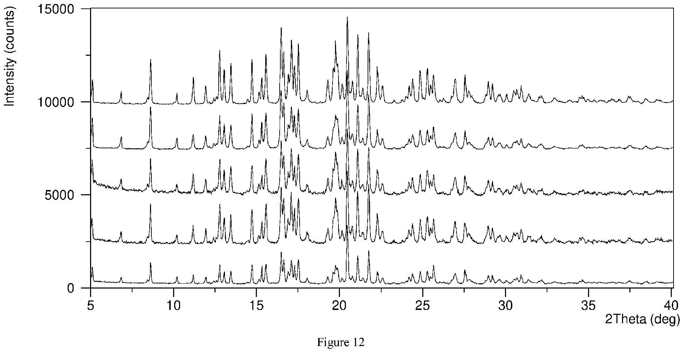

An appropriate amount of Form CSII of the present disclosure were stored under different conditions of 25 °C/60%RH, 40 °C/75%RH and 60 °C/75%RH. Chemical purity and crystalline form were tested by HPLC and XRPD respectively. The results are shown in Table 14, and the XRPD overlay is shown in Figure 12. The results show that Form CSII is stable after storage under 25 °C/60%RH condition for at least 9 months, indicating Form CSII has good stability under long-term condition. Form CSII is stable after storage under 40 °C/75%RH condition for at least 6 months, indicating Form CSII has good stability under accelerated condition. Form CSII is stable after storage under 60 °C/75%RH condition for at least 1 month, indicating Form CSII has good stability under stress condition.

**Table 14**

| Initial form | Conditions | Packing conditions | Time | Solid form | Purity |
|---|---|---|---|---|---|
| Form CSII | Initial | N/A | - | Form CSII | 99.96% |
| | 25 °C/60%RH | Sealed with desiccants | 9 months | Form CSII | 99.95% |
| | | Open | 9 months | Form CSII | 99.90% |
| | 40 °C/75%RH | Sealed with desiccants | 6 months | Form CSII | 99.90% |
| | 60 °C/75%RH | Sealed with desiccants | 1 month | Form CSII | 99.94% |

Open: the sample was placed into a glass vial, and the vial was covered by aluminum foil with holes.

Sealed with desiccants: the sample was placed into a glass vial, and the vial was covered by aluminum foil with holes, then the vial was sealed into an aluminum foil bag with 1 g of silica gel desiccant.

### Example 14 Stability of Form CSII upon mechanical force

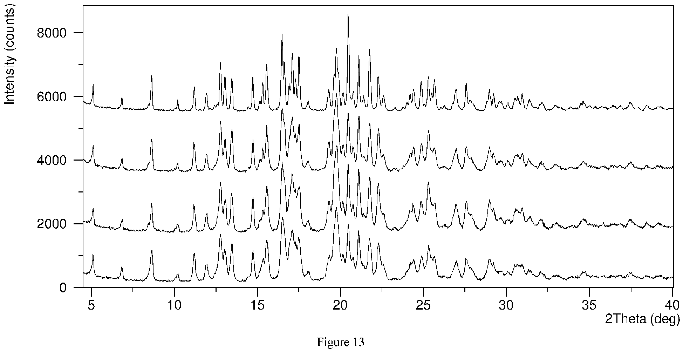

An appropriate amount of Form CSII was compressed into tablets under different pressures with suitable tableting die. Crystalline form before and after tableting were tested by XRPD. The test results are shown in Table 15, and the XRPD overlay is shown in Figure 13. The results show that Form CSII has good stability under different pressures.

**Table 15**

| Before tableting | Pressure | After tableting |
|---|---|---|
| Form CSII | 5 kN | Form CSII |
| | 10 kN | Form CSII |
| | 20 kN | Form CSII |

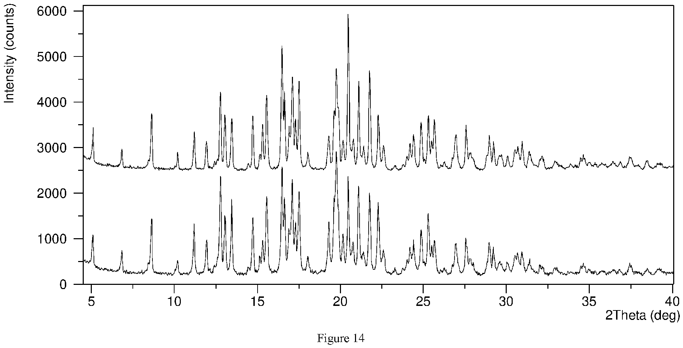

Form CSII was milled for 5 minutes at a vibration speed of 500 rpm by a ball mill. Crystalline form before and after ball milling were tested by XRPD, and the XRPD overlay is shown in Figure 14. The results show that no form change of Form CSII is observed after ball milling, and Form CSII has good stability.

### Example 15 Purification ability of Form CSII

Form CSII of the present invention was prepared using the starting material with a purity of 99.14%. HPLC was applied to test the chemical purity of starting material and Form CSII, and the results are listed in Table 16. The results show that Form CSII has good purification ability.

**Table 16**

| Solid form | Chemical purity | Purity increment |
|---|---|---|
| Starting material | 99.14% | - |
| Form CSII | 99.79% | 0.65% |

### Example 16 Preparation of Form CSII drug product

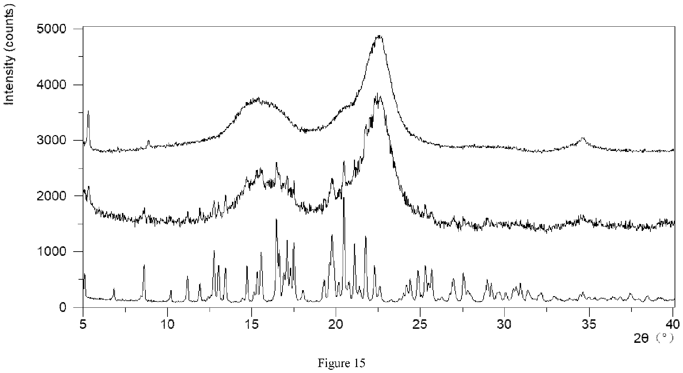

Form CSII drug product was prepared as the formulation shown in Table 17 and preparation process shown in Table 18. The blank formulation was shown in Table 19. The blank mixed powder, the samples before and after the formulation were tested by XRPD, and the results are shown in Figure 15. The results show that Form CSII is stable before and after the formulation process.

**Table 17**

| No. | Component | mg/unit | % (w/w) |
|---|---|---|---|
| 1 | Co-crystal of Compound I hydrochloride and L-tartaric acid (Form CSII) | 14.0 | 14.0% |
| 2 | Microcrystalline cellulose | 74.25 | 74.3% |
| 3 | Hypromellose | 3.0 | 3.0% |
| 4 | Crospovidone | 6.0 | 6.0% |
| 5 | Micro powder silicone | 0.5 | 0.5% |
| 6 | Magnesium stearate | 1.0 | 1.0% |
| Subtotal | | 98.75 | 98.8% |
| 7 | Crospovidone | 1.0 | 1.0% |
| 8 | Magnesium stearate | 0.25 | 0.3% |
| Total | | 100 | 100.0% |
| Note: 14 mg of co-crystal of Compound I hydrochloride and L-tartaric acid (Form CSII) is equivalent to 10 mg of Compound I. | | | |

**Table 18**

| Stage | Procedure |
|---|---|
| Pre-blending | According to the formulation, materials No. 1-6 were weighed into an LDPE bags and manually blended for 2 minutes. |
| Simulation of dry granulation | The mixture (500 mg ± 10 mg) was pressed by a ENERPAC single punch manual tablet press with the die ϕ 20 mm round tooling under the pressure of 5 ± 1 kN. Then the obtained flakes were pulverized and sieved through a 20-mesh sieve. |
| Blending | The mixed powder obtained by simulating dry granulation and the weighed additional excipients 7 and 8 were added to the above LDPE bag and manually blended for 2 minutes to obtain the final mixed powder. |
| Capsule filling | About 100 mg of the final mixed powder was weighed and filled into the 2# capsule shell. |
| Package | 1 capsule with 1 g of desiccant was placed in a 35 cc HDPE bottle and then sealed. |

**Table 19**

| No. | Component | mg/unit | % (w/w) |
|---|---|---|---|
| 1 | Microcrystalline cellulose | 74.25 | 74.3% |
| 2 | Hypromellose | 3.0 | 3.0% |
| 3 | Crospovidone | 7.0 | 7.0% |
| 4 | Micro powder silicone | 0.5 | 0.5% |
| 5 | Magnesium stearate | 1.25 | 1.3% |
| Total | | 86 | 86.1% |

### Example 17 Dissolution profile of Form CSII drug product

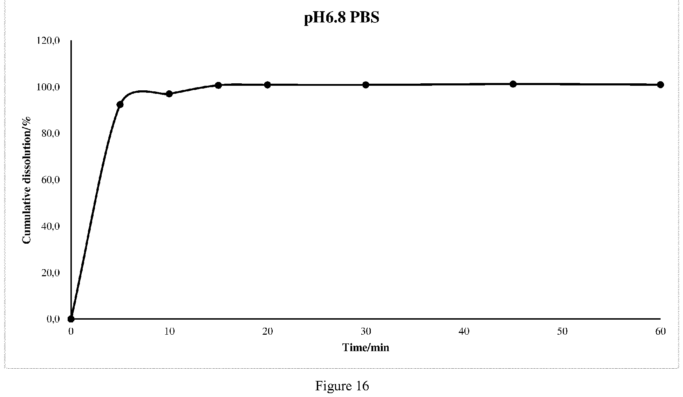

Dissolution test was performed on Form CSII drug product obtained from Example 16. Dissolution method according to Chinese Pharmacopoeia 2020<0931> was used. The test conditions are shown in Table 20. Dissolution results are presented in Table 21 and the dissolution curve is shown in Figure 16, which indicate that Form CSII drug product possesses favorable dissolution.

**Table 20**

| | |
|---|---|
| Dissolution apparatus | Agilent 708-DS |
| Method | Paddle + sinker |
| Volume | 900 mL |
| Speed | 50 rpm |
| Temperature | 37 °C |
| Sampling point | pH 6.8 PBS: 5, 10, 15, 20, 30, 45, 60 min |

**Table 21**

| Time (min) | Cumulative drug release (%) |
|---|---|
| | Form CSII |
| 0 | 0.0 |
| 5 | 92.4 |
| 10 | 97.0 |
| 15 | 100.7 |
| 20 | 100.9 |
| 30 | 100.9 |
| 45 | 101.2 |
| 60 | 101.0 |

### Example 18 Stability of Form CSII drug product

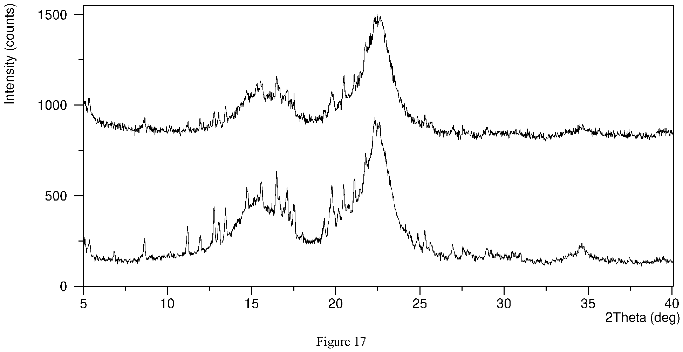

Form CSII drug product was sealed with 1 g of desiccant and stored under 25 °C/60%RH condition. Purity and crystalline form of the sample were tested by HPLC and XRPD respectively to check the stability of Form CSII drug product. The results are shown in Table 22 and the XRPD overlay is shown in Figure 17. The results indicate that Form CSII drug product is stable after storage under 25 °C/60%RH condition for at least 1 month.

**Table 22**

| Initial form | Initial purity | Time | Solid form | Purity |
|---|---|---|---|---|
| Form CSII | 99.97% | 1 month | Form CSII | 99.97% |

### Example 19 Flowability of Form CSIII

About 500 mg of Form CSIII or Form I in the prior art was weighed into the 5-mL measuring cylinder gently to avoid vibration and the volume before tapped was recorded. The bulk density ρ₀ was calculated with the formula "bulk density ρ₀ = powder mass/volume before tapped". Then the sample was tapped for 1250 times by ZS-2E tap density tester and the tapped volume was recorded. The tapped density ρ_{f} was calculated with the formula "tapped density ρ_{f} =powder mass/volume after tapped". The degree of compression (c), also known as Compressibility index or Carr index, was calculated based on the measured bulk density and tap density of the sample with the formula c = (ρ_{f}-ρ₀)/p_{f}*100%. Criteria of flowability according to ICH Q4B Annex 13 is shown in Table 23.

**Table 23**

| Compressibility index (%) | Flowability |
|---|---|
| ≦ 10 | Excellent |
| 11-15 | Good |
| 16-20 | Fair |
| 21-25 | Passable |
| 26-31 | poor |
| 32-37 | Very poor |
| >38 | Very, very poor |

Flowability evaluation results of Form CSIII and Form I in the prior art are presented in Table 24, which indicate that flowability of Form CSIII is remarkably superior to that of Form I in the prior art.

**Table 24**

| Solid form | Compressibility index | Flowability |
|---|---|---|
| Form I in the prior art | 29% | Poor |
| Form CSIII | 17% | Fair |

### Example 20 Compressibility of Form CSIII

The samples were pressed by the ENERPAC single punch manual tablet press. 80 mg of Form CSIII and Form I in the prior art were added into dies of a Φ6mm round tooling, and compressed into tablets under a pressure of 3 kN, respectively. The elastic recovery was completed after storage at room temperature for 24 hours, then the diameter (D) and thickness (L) of the tablet was measured by a caliper, and the hardness (H) was tested with a tablet hardness tester. Tensile strength of the powder was calculated with the following formula: T=2H/πDL. Under a certain force, the greater the tensile strength, the better the compressibility. The results are listed in Table 25. The results indicate Form CSIII has better compressibility compared with Form I in the prior art.

**Table 25**

| Solid form | Tensile strength (MPa) |
|---|---|
| Form I in the prior art | 0.552 |
| Form CSIII | 0.707 |

### Example 21 Solubility of Form CSIII

About 3-5 mg of Form CSIII or Form I in the prior art was weighed into each vial respectively, and 0.05 mL of corresponding solvent was added step-wise till the solid was totally dissolved. The weight of solid (m), the undissolved volume (v₁) and the dissolved volume (v₂) were recorded respectively. According to the formula: m/v₂<S<m/v₁, which m needs to be converted from the weighed mass to the corresponding mass of Compound I, the solubility (S) results are calculated and shown in Table 26.

The results show that in common alcohol, ketone, ester and ether solvents, the solubility of Form CSIII is higher than that of Form I in the prior art, especially in tetrahydrofuran, the solubility of Form CSIII is at least 13 times that of Form I in the prior art.

**Table 26**

| Solvent | Solubility of Form I in the prior art (mg/mL) | Solubility of Form CSIII (mg/mL) |
|---|---|---|
| n-Propanol | 6.3<S<12.5 | S>18.0 |
| Acetone | 1.5<S<2.2 | S>17.0 |
| Ethyl acetate | S<1.5 | 2.1<S<2.7 |
| Tetrahydrofuran | S<1.5 | S>20.0 |

### Example 22 Light stability of Form CSIII

An appropriate amount of Form CSIII of the present disclosure was placed in a light stability chamber with the following setting values: light: 8 k Lux, UV: 1.5 w/m². The chemical purity and crystalline form were tested by HPLC and XRPD respectively, and the results are shown in Table 27. The results show that Form CSIII has good light stability.

**Table 27**

| Initial form | Initial purity | Time | Solid form | Purity |
|---|---|---|---|---|
| Form CSIII | 99.83% | 2 weeks | Form CSIII | 99.76% |

### Example 23 Stability of Form CSIII

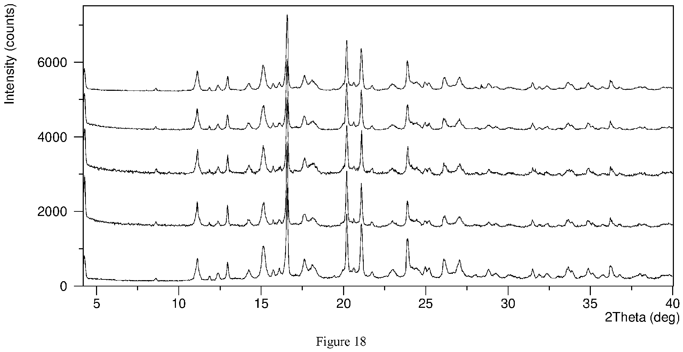

An appropriate amount of Form CSIII of the present disclosure were stored under different conditions of 25 °C/60%RH, 40 °C/75%RH and 60 °C/75%RH. Chemical purity and crystalline form were tested by HPLC and XRPD respectively. The results are shown in Table 28, and the XRPD overlay is shown in Figure 18. The results show that Form CSIII is stable after storage under 25 °C/60%RH condition for at least 9 months, indicating Form CSIII has good stability under long-term condition. Form CSIII is stable after storage under 40 °C/75%RH condition for at least 3 months, indicating Form CSIII has good stability under accelerated condition. Form CSIII is stable after storage under 60 °C/75%RH condition for at least 2 months, indicating Form CSIII has good stability under stress condition.

**Table 28**

| Initial form | Conditions | Packing conditions | Time | Solid form | Purity |
|---|---|---|---|---|---|
| Form CSIII | Initial | N/A | - | Form CSIII | 99.86% |
| | 25 °C/60%RH | Sealed with desiccants | 9 months | Form CSIII | 99.92% |
| | | Open | 9 months | Form CSIII | 99.91% |
| | 40 °C/75%RH | Sealed with desiccants | 3 months | Form CSIII | 99.91% |
| | 60 °C/75%RH | Sealed with desiccants | 2 months | Form CSIII | 99.86% |

Open: the sample was placed into a glass vial, and the vial was covered by aluminum foil with holes.

Sealed with desiccants: the sample was placed into a glass vial, and the vial was covered by aluminum foil with holes, then the vial was sealed into an aluminum foil bag with 1 g of silica gel desiccant.

### Example 24 Stability of Form CSIII upon mechanical force

An appropriate amount of Form CSIII was compressed into tablets under different pressures with suitable tableting die. Crystalline form before and after tableting were tested by XRPD. The test results are shown in Table 29. The results show that Form CSIII has good stability under different pressures.

**Table 29**

| Before tableting | Pressure | After tableting |
|---|---|---|
| Form CSIII | 5 kN | Form CSIII |
| | 10 kN | Form CSIII |
| | 20 kN | Form CSIII |

Form CSIII was milled for 5 minutes at a vibration speed of 500 rpm by a ball mill. Crystalline form before and after ball milling were tested by XRPD. The results show that no form change of Form CSIII is observed after ball milling, and Form CSIII has good stability.

### Example 25 Purification ability of Form CSIII

Form CSIII of the present invention was prepared using the starting material with a purity of 99.14%. HPLC was applied to test the chemical purity of starting material and Form CSIII, and the results are listed in Table 30. The results show that Form CSIII has good purification ability.

**Table 30**

| Solid form | Chemical purity | Purity increment |
|---|---|---|
| Starting material | 99.14% | - |
| Form CSIII | 99.86% | 0.72% |

### Example 26 Preparation of CSIII drug product

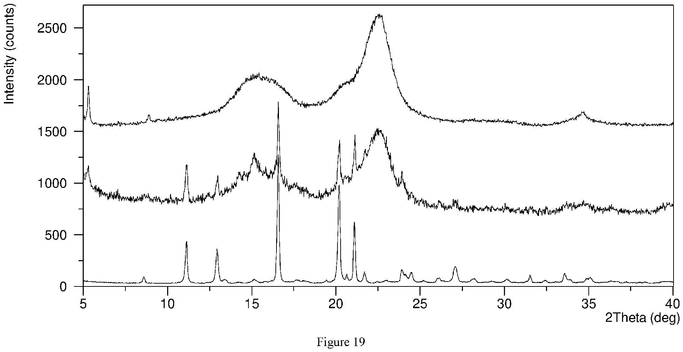

Form CSIII drug product was prepared as the formulation shown in Table 31 and preparation process shown in Table 18. The blank formulation was shown in Table 19. The blank mixed powder, the samples before and after the formulation were tested by XRPD, and the results are shown in Figure 19. The results show that Form CSIII is stable before and after the formulation process.

**Table 31**

| No. | Component | mg/unit | % (w/w) |
|---|---|---|---|
| 1 | Co-crystal of Compound I hydrochloride and citric acid (Form CSIII) | 18.1 | 18.1% |
| 2 | Microcrystalline cellulose | 70.15 | 70.2% |
| 3 | Hypromellose | 3.0 | 3.0% |
| 4 | Crospovidone | 6.0 | 6.0% |
| 5 | Micro powder silicone | 0.5 | 0.5% |
| 6 | Magnesium stearate | 1.0 | 1.0% |
| Subtotal | | 98.75 | 98.8% |
| 7 | Crospovidone | 1.0 | 1.0% |
| 8 | Magnesium stearate | 0.25 | 0.3% |
| Total | | 100 | 100.0% |
| Note: 18.0 mg of co-crystal of Compound I hydrochloride and citric acid (Form CSIII) is equivalent to 10 mg of free base of Compound I. | | | |

### Example 27 Dissolution profile of Form CSIII drug product

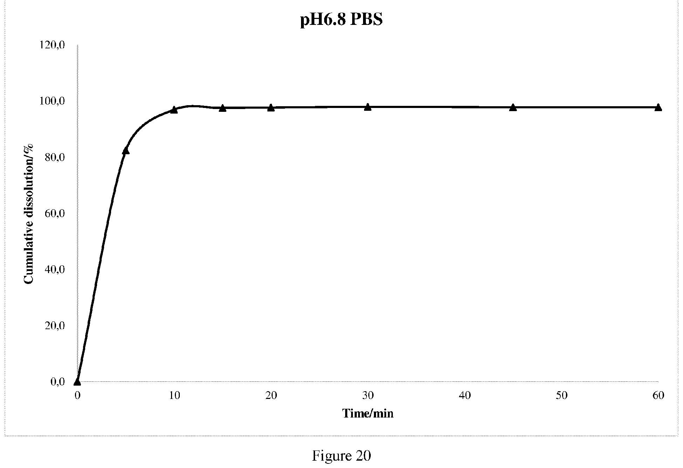

Dissolution test was performed on Form CSIII drug product obtained from Example 26. Dissolution method according to Chinese Pharmacopoeia 2020<0931> was used. The test conditions are as Table 20. Dissolution results are presented in Table 32 and the dissolution curve is shown in Figure 20, which indicate that Form CSIII drug product possesses favorable dissolution.

**Table 32**

| Time (min) | Cumulative drug release (%) |
|---|---|
| 0 | 0.0 |
| 5 | 82.4 |
| 10 | 96.9 |
| 15 | 97.5 |
| 20 | 97.7 |
| 30 | 97.9 |
| 45 | 97.8 |
| 60 | 97.8 |

### Example 28 Stability of Form CSIII in drug product

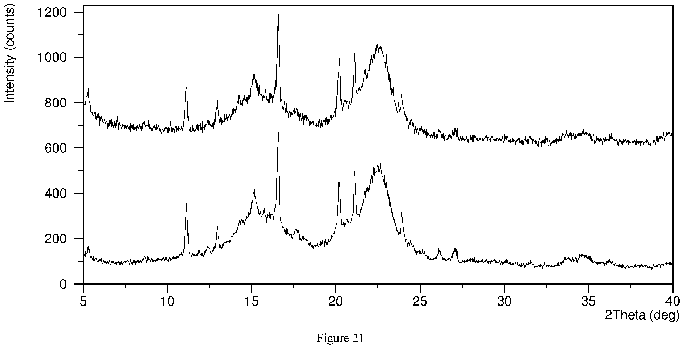

Form CSIII drug product was sealed with 1 g of desiccant and stored under 25 °C/60% RH condition. Purity and crystalline form of the sample were tested by HPLC and XRPD respectively to check the stability of Form CSIII drug product. The results are shown in Table 33 and the XRPD overlay is shown in Figure 21. The results indicate that Form CSIII drug product is stable after storage under 25 °C/60% RH condition for at least 1 month.

**Table 33**

| Initial form | Initial purity | Time | Solid form | Purity |
|---|---|---|---|---|
| Form CSIII | 99.91% | 1 month | Form CSIII | 99.91% |

### Example 29 Flowability of Form CSIV

About 500 mg of Form CSIV or Form I in the prior art was weighed into the 5-mL measuring cylinder gently to avoid vibration and the volume before tapped was recorded. The bulk density ρ₀ was calculated with the formula "bulk density ρ₀ = powder mass/volume before tapped". Then the sample was tapped for 1250 times by ZS-2E tap density tester and the tapped volume was recorded. The tapped density ρ_{f} was calculated with the formula "tapped density ρ_{f} =powder mass/volume after tapped". The degree of compression (c), also known as Compressibility index or Carr index, was calculated based on the measured bulk density and tap density of the sample with the formula c = (ρ_{f}-ρ₀)/ρ_{f}* 100%. Criteria of flowability according to ICH Q4B Annex 13 is shown in Table 23.

Flowability evaluation results of Form CSIV and Form I in the prior art are presented in Table 34, which indicate that flowability of Form CSIV is remarkably superior to that of Form I in the prior art.

**Table 34**

| Solid form | Compressibility index | Flowability |
|---|---|---|
| Form I in the prior art | 29% | Poor |
| Form CSIV | 9.1% | Excellent |

### Example 30 Compressibility of Form CSIV

The samples were pressed by the ENERPAC single punch manual tablet press. 80 mg of Form CSIV and Form I in the prior art were added into dies of a Φ6mm round tooling, and compressed into tablets under a pressure of 3 kN, respectively. The elastic recovery was completed after storage at room temperature for 24 hours, then the diameter (D) and thickness (L) of the tablet was measured by a caliper, and the hardness (H) was tested with a tablet hardness tester. Tensile strength of the powder was calculated with the following formula: T=2H/πDL. Under a certain force, the greater the tensile strength, the better the compressibility. The results are listed in Table 35. The results indicate Form CSIV has better compressibility compared with Form I in the prior art.

**Table 35**

| Solid form | Tensile strength (MPa) |
|---|---|
| Form I in the prior art | 0.552 |
| Form CSIV | 0.705 |

### Example 31 Light stability of Form CSIV

An appropriate amount of Form CSIV of the present disclosure was placed in a light stability chamber with the following setting values: light: 8 k Lux, UV: 1.5 w/m². The chemical purity and crystalline form were tested by HPLC and XRPD respectively, and the results are shown in Table 36. The results show that Form CSIV has good light stability.

**Table 36**

| Initial form | Initial purity | Time | Solid form | Purity |
|---|---|---|---|---|
| Form CSIV | 99.92 | 2 weeks | Form CSIV | 99.92% |

### Example 32 Stability of Form CSIV

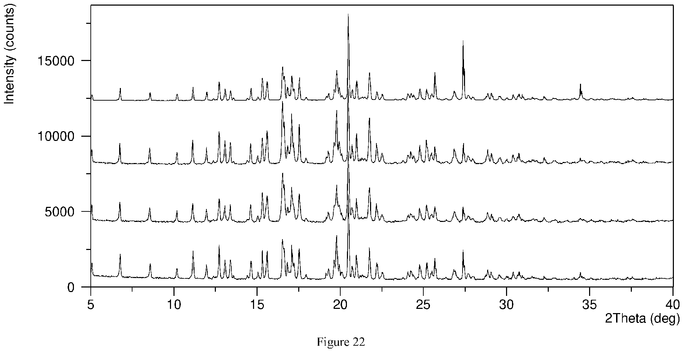

An appropriate amount of Form CSIV of the present disclosure were stored under different conditions of 25 °C/60%RH, 40 °C/75%RH and 60 °C/75%RH. Chemical purity and crystalline form were test by HPLC and XRPD respectively. The results are shown in Table 37, and the XRPD overlay is shown in Figure 22. The results show that Form CSIV is stable after storage under 25 °C/60%RH condition for at least 6 months, indicating Form CSIV has good stability under long-term condition. Form CSIV is stable after storage under 40 °C/75%RH condition for at least 6 months, indicating Form CSIV has good stability under accelerated condition. Form CSIV is stable after storage under 60 °C/75%RH condition for at least 2 weeks, indicating Form CSIV has good stability under stress condition.

**Table 37**

| Initial form | Conditions | Packing conditions | Time | Solid form | Purity |
|---|---|---|---|---|---|
| Form CSIV | Initial | N/A | - | Form CSIV | 99.95% |
| | 25 °C/60%RH | Sealed with desiccants | 6 months | Form CSIV | 99.92% |
| | 40 °C/75%RH | Sealed with desiccants | 6 months | Form CSIV | 99.93% |
| | 60 °C/75%RH | Sealed with desiccants | 2 weeks | Form CSIV | 99.98% |

Sealed with desiccants: the sample was placed into a glass vial, and the vial was covered by aluminum foil with holes, then the vial was sealed into an aluminum foil bag with 1 g of silica gel desiccant.

### Example 33 Stability of Form CSIV upon mechanical force

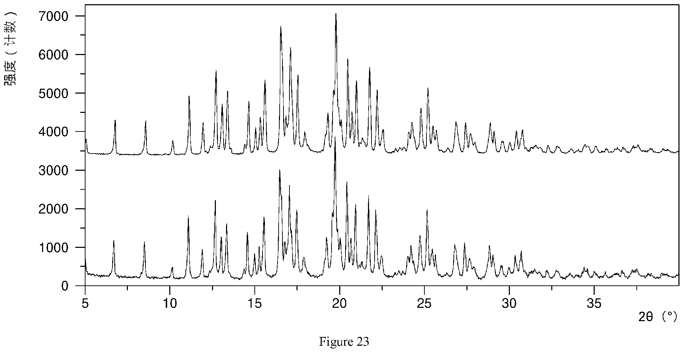

Form CSIV was milled for 5 minutes at a vibration speed of 500 rpm by a ball mill. Crystalline form before and after ball milling were tested by XRPD, and the XRPD overlay is shown in Figure 23. The results show that no form change of Form CSIV is observed after ball milling, and Form CSIV has good stability.

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A tartaric acid co-crystal of Compound I hydrochloride

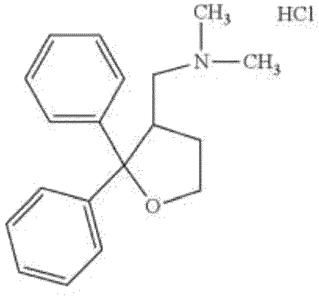

2. The tartaric acid co-crystal according to claim 1 is Form CSII, wherein the X-ray powder diffraction pattern comprises one or two or three characteristic peaks at 2theta values of 8.6°±0.2°, 12.8°±0.2° and 20.5°±0.2° using Cu-Kα radiation.

3. The Form CSII according to claim 2, wherein the X-ray powder diffraction pattern comprises one or two or three characteristic peaks at 2theta values of 14.7°±0.2°, 21.1°±0.2° and 21.8°±0.2° using Cu-Kα radiation.

4. The Form CSII according to claim 2, wherein the X-ray powder diffraction pattern comprises one or two or three characteristic peaks at 2theta values of 13.5°±0.2°, 15.6°±0.2° and 17.5°±0.2° using Cu-Kα radiation.

5. The Form CSII according to claim 3, wherein the X-ray powder diffraction pattern comprises one or two or three characteristic peaks at 2theta values of 13.5°±0.2°, 15.6°±0.2° and 17.5°±0.2° using Cu-Kα radiation.

6. The Form CSII according to claim 2, wherein the X-ray powder diffraction pattern is substantially as depicted in Figure 1 or Figure 4 using Cu-Kα radiation.

7. The tartaric acid co-crystal according to claim 1, wherein tartaric acid is L-tartaric acid.

8. The Form CSII according to claim 2, wherein Form CSII is an anhydrate.

9. A citric acid co-crystal of Compound I hydrochloride

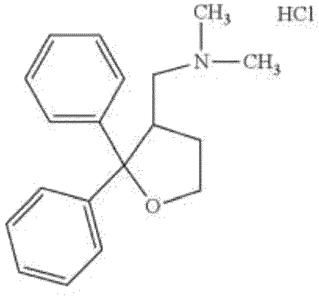

10. The citric acid co-crystal according to claim 9 is Form CSIII, wherein the X-ray powder diffraction pattern comprises one or two or three characteristic peaks at 2theta values of 16.6°±0.2°, 20.2°±0.2° and 21.1°±0.2° using Cu-Kα radiation.

11. The Form CSIII according to claim 10, wherein the X-ray powder diffraction pattern comprises one or two or three characteristic peaks at 2theta values of 4.3°±0.2°, 11.1°±0.2° and 12.9°±0.2° using Cu-Kα radiation.

12. The Form CSIII according to claim 10, wherein the X-ray powder diffraction pattern is substantially as depicted in Figure 7 using Cu-Kα radiation.

13. The Form CSIII according to claim 10, wherein Form CSIII is an anhydrate.

14. A malic acid co-crystal of Compound I hydrochloride

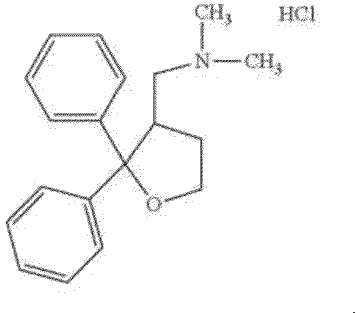

15. The malic acid co-crystal according to claim 14 is Form CSIV, wherein the X-ray powder diffraction pattern comprises one or two or three characteristic peaks at 2theta values of 8.6°±0.2°, 20.5°±0.2° and 21.8°±0.2° using Cu-Kα radiation.

16. The Form CSIV according to claim 15, wherein the X-ray powder diffraction pattern comprises at least one characteristic peak at 2theta values of 12.7°±0.2°, 11.1°±0.2° and 17.5°±0.2° using Cu-Kα radiation.

17. The Form CSIV according to claim 15, wherein the X-ray powder diffraction pattern comprises at least one characteristic peak at 2theta values of 6.8°±0.2°, 10.2°±0.2° and 21.0°±0.2° using Cu-Kα radiation.

18. The Form CSIV according to claim 16, wherein the X-ray powder diffraction pattern comprises at least one characteristic peak at 2theta values of 6.8°±0.2°, 10.2°±0.2° and 21.0°±0.2° using Cu-Kα radiation.

19. The Form CSIV according to claim 15, wherein the X-ray powder diffraction pattern is substantially as depicted in Figure 8 or Figure 10 using Cu-Kα radiation.

20. The malic acid co-crystal according to claim 14, wherein malic acid is DL- malic acid.

21. The Form CSIV according to claim 15, wherein Form CSIV is an anhydrate.

22. A pharmaceutical composition, wherein said pharmaceutical composition comprises a therapeutically effective amount of Form CSII according to claim 2, Form CSIII according to claim 10, Form CSIV according to claim 15, or mixture thereof, and pharmaceutically acceptable excipients.
